# EUROPEAN PATENT SPECIFICATION

(11) **EP 4 323 388 B1**
(45) Date of publication and mention of the grant of the patent: **29.01.2025**
(21) Application number: 22715644.5
(22) Date of filing: 15.04.2022
(51) Int. Cl.: C07K 14/47, A61K 38/00, A61K 47/64, C07K 14/00

(54) **N-TERMINAL CAPPING MODULES OF ANKYRIN REPEAT DOMAINS**
VERBESSERTE N-TERMINALE KAPPUNGSMODULE VON ANKYRINWIEDERHOLUNGSDOMÄNEN
MODULES DE COIFFAGE DE DOMAINES DE RÉPÉTITION D'ANKYRINE À N-TERMINAUX AMÉLIORÉS

(30) Priority: 16.04.2021 US 202117232470; 17.08.2021 WO PCT/EP2021/072819; 28.09.2021 EP 21199643
(43) Date of publication of application: 21.02.2024
(73) Proprietor: Athebio AG, 8952 Zurich-Schlieren (CH)
(72) Inventor: EAPEN, Rohan Sakariah, 8952 Zurich-Schlieren (CH); FORRER, Patrik, 8952 Zurich-Schlieren (CH)
(74) Representative: Hutter, Bernd
(86) International application number: PCT/EP2022/060178
(87) International publication number: WO 2022/219185

(56) References cited:
- WO-A1-2014/191574
- WO-A1-2020/245746
- WO-A2-2012/069655
- WO-A2-2016/023898
- WO-A2-2020/190852
- ANDREAS PLÜCKTHUN: "Designed Ankyrin Repeat Proteins (DARPins): Binding Proteins for Research, Diagnostics, and Therapy", ANNUAL REVIEW OF PHARMACOLOGY AND TOXICOLOGY, vol. 55, no. 1, 6 January 2015 (2015-01-06), pages 489 - 511, XP055217451, ISSN: 0362-1642, DOI: 10.1146/annurev-pharmtox-010611-134654
- SCHILLING JOHANNES ET AL: "Thermostable designed ankyrin repeat proteins (DARPins) as building blocks for innovative drugs", JOURNAL OF BIOLOGICAL CHEMISTRY, 1 November 2021 (2021-11-01), US, pages 101403, XP055866606, ISSN: 0021-9258, DOI: 10.1016/j.jbc.2021.101403

## Description

### Field of the invention

The present invention relates to an ankyrin repeat domain and related products and methods. In particular, the present invention relates to an ankyrin repeat domain with L at position 24 of the N-terminal capping module.

### Background of the invention

Different classes of specific binding proteins have evolved in nature, the most widely known class being immunoglobulins of vertebrates. Another class of specific binding proteins are repeat proteins. Similar to the role that immunoglobulins play in vertebrates, repeat proteins were found to be involved in the adaptive immune system of jawless fish. However, repeat proteins play a much wider role beyond this function and mediate protein-protein interactions across all phyla to fulfill diverse biological functions. In fact, they constitute the largest group of natural proteins mediating specific binding (e.g. reviewed in Forrer, P., et al., FEBS letters 539, 2-6, 2003). Repeat proteins bind their targets via the repeat domain, which is made up of a variable number of repeats that stack on each other through their conserved interfaces to create the compactly folded repeat domain. Specific target binding is then achieved through variable residues on the surface of the repeat domain (Forrer 2003, loc. cit. and WO 2002/020565).

Ankyrin repeat proteins are a well-studied class of repeat proteins. The ankyrin repeat usually comprises 33 amino acid residues forming two antiparallel alpha-helices and a beta-turn. The folded ankyrin repeat domain comprising the stacked ankyrin repeats has a right-handed solenoid structure with a compact hydrophobic core and a large binding surface, which allows it to adapt to its respective binding partners (e.g. Binz, H.K., et al., Nat. Biotechnol. 22, 575-582, 2004).

Plückthun and coworkers derived a consensus sequence motif from naturally occurring ankyrin repeats (e.g., Binz, H.K., et al., J. Mol. Biol., 332, 489-503, 2003 and WO 2002/020565). The derived ankyrin repeat consensus motif is 33-amino acid residues long and comprises fixed and variable positions (the latter also being referred to as randomized positions). The fixed positions correspond mainly to framework residues that are primarily responsible for the structural integrity of the ankyrin repeat domain, in particular, for the interrepeat stacking interactions. The variable positions correspond to surface-exposed residues that do not strongly contribute to the structural integrity of the ankyrin repeat domain but are potentially involved in target binding (though surface-exposed framework residues may be involved in target binding too).

Libraries of proteins, each having an ankyrin repeat domain with internal ankyrin repeats that were based on such ankyrin repeat consensus motif, were then created (Binz, 2004, loc. cit.). Certain variable positions of the consensus motif were randomized in each internal ankyrin repeat to allow for the binding to different targets, thereby creating the diversity of the library. In order to avoid aggregation of ankyrin repeat domains consisting only of internal ankyrin repeats, the internal ankyrin repeats were flanked by an N-terminal capping module and a C-terminal capping module to shield the hydrophobic core of the domain from the solvent (Forrer, 2003, loc. cit. and Binz, 2003, loc. cit.). These capping modules were based on the capping repeats of the murine guanine-adenine-binding protein (GA-binding protein).

Using such a synthetic library of designed ankyrin repeat proteins (DARPins), DARPins against specific targets can be selected with common selection methods, including phage display, ribosome display and yeast display, and were shown to have favorable properties. While displaying binding specificities and affinities that are comparable to immunoglobulins, DARPins are much more robust and can be easily engineered into multispecific binding proteins that are easily expressed and purified (e.g. Plückthun, A., Annu. Rev. Pharmacol. Toxicol. 55, 489-511, 2015).

Following the design of the original DARPin library by Plückthun and coworkers (Binz, 2003, loc. cit. and WO 2002/020565), it was shown that specific mutations in the N-terminal capping module can increase the thermostability of an ankyrin repeat domain (WO 2012/069655 and PCT/EP2021/072819).

There remains a need to further improve the properties of proteins comprising an ankyrin repeat domain, such as the thermostability of the ankyrin repeat domain.

### Summary of the invention

The present invention is based on the discovery that specific mutations in the N-terminal capping module can significantly improve the properties of an ankyrin repeat domain. In particular, it has been found that the amino acid residue at position 24 of the N-terminal capping module is of key importance for the thermostability of the ankyrin repeat domain. A leucine at this position has been found to be particularly favorable for the thermostability of the ankyrin repeat domain. Furthermore, the effects of this mutation were transferable to ankyrin repeat domains with diverging sequences, demonstrating the general importance of this position in the N-terminal capping module for the thermostability of ankyrin repeat domains.

Accordingly, the present invention provides a protein comprising an ankyrin repeat domain, wherein the ankyrin repeat domain has an N-terminal capping module with L at position 24.

In further aspects, the present invention provides a protein library comprising such protein and a method of selection using such protein library.

The present invention also provides a pharmaceutical composition comprising the protein of the invention, a nucleic acid encoding it and a vector or cell comprising such nucleic acid.

In a further aspect, the present invention provides a method of preparing a protein of the invention comprising culturing a cell having a nucleic acid encoding the protein under conditions allowing expression thereof and then purifying the expressed protein.

In a further aspect, the present invention relates to the protein of the invention for use in a method of treatment.

Related products and methods are also provided, as will be apparent from the following detailed description.

### Brief Description of the Figures

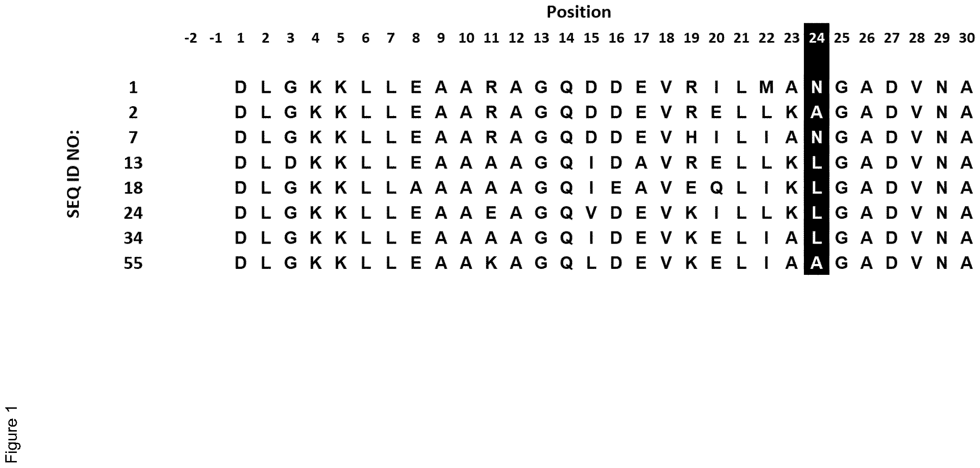

**Figure 1****:** Alignment of different N-terminal capping module sequences from the sequence listing. Position 24 of each N-terminal capping module is highlighted.
**Figure 2****:** Thermal stability of the designed ankyrin repeat proteins P#59 and P#63.

Traces from thermal denaturation of P#59 and P#63 are shown. The thermal denaturation is followed by the CD signal at 222 nm in PBS, 2M GdmCl, pH 7.4. The Tm values for P#59 and P#63 were determined to be 67.6°C and 61.3°C, respectively.

FF, fraction folded in %; T, temperature in °C.

### Definitions

"A", "an", and "the" include plural reference unless the context clearly dictates otherwise. Thus, for example, reference to a protein comprising an ankyrin repeat domain refers to one or more such proteins.

The amino acid residues are referred to herein interchangeably by their full name, their three-letter code or their one-letter code. The "naturally occurring amino acid residues" refer to the twenty amino acid residues that are most commonly found in nature, i.e. A, R, N, D, C, E, Q, G, H, I, L, K, M, F, P, S, T, W, Y and V.

An "ankyrin repeat" refers to a short sequence of amino acid residues forming a structural motif. Ankyrin repeats occur in consecutive copies, are involved in protein-protein interactions and the core of the ankyrin repeat forms a helix-loop-helix structure (e.g.,SMART accession number: SM00248).

The term "ankyrin repeat domain" refers to a protein domain comprising an N-terminal capping module, a C-terminal capping module and one or more ankyrin repeats in between (also referred to as "internal ankyrin repeats"). The folded ankyrin repeat domain has a right-handed solenoid structure with a large binding surface that is adaptable to specifically bind targets. The ankyrin repeat domain is generally very robust and can sustain a significant number of mutations, including substitutions, additions and deletions, without destroying its overall structure or function. The residues that contribute to the structural integrity of the ankyrin repeat domain, including the interrepeat interactions, are referred to as "framework residues", whereas the residues that contribute to target binding, either through direct interaction with the target or by influencing residues that directly interact with the target, e.g., by stabilizing them, are referred to as "target interaction residues". A single amino acid residue can be both - a framework and a target interaction residue - at the same time and framework residues and target interaction residues may be found not only in the internal ankyrin repeats, but also the N-terminal capping module and/or the C-terminal capping module.

The internal ankyrin repeats contribute to the structural stability of the ankyrin repeat domain through their stacking interactions with the neighboring repeats. An internal ankyrin repeat often consists of 33 amino acid residues.

The capping modules have a hydrophobic inside surface that is suitable for interacting with the adjacent internal ankyrin repeat and a hydrophilic outside surface to shield the hydrophobic core from the solvent. In some embodiments, the N-terminal capping module and/or the C-terminal capping module are a N-terminal capping repeat and/or C-terminal capping repeat, respectively, which have a similar or the same fold as the adjacent internal ankyrin repeat(s) and/or sequence similarities to said adjacent internal ankyrin repeat(s).

The terms "binding", "specific binding" or the like when used in reference to a target mean a binding interaction that is measurably different from a non-specific interaction, e.g., the interaction with a control molecule that is unrelated to the specific target. Control molecules that are commonly used to measure such non-specific interaction include bovine serum albumin, bovine casein and *Escherichia coli* (*E. coli*) maltose binding protein. In certain instances, the terms "binding", "specific binding" or the like mean that only the target is bound and substantially no other molecule. Specific binding can be determined, for instance, by measuring the dissociation constant (Kd) for the target and/or by comparing the binding to the target with the binding to a control molecule. The Kd can be measured by various conventional techniques, such as isothermal titration calorimetry, radioligand binding assay, fluorescence resonance energy transfer, and surface plasmon resonance. The binding specificity is generally measured in standardized solutions, such as PBS. For instance, the Kd for the target in PBS is at least 10, at least 10², at least 10³ or at least 10⁴ times lower than the corresponding Kd for a control molecule that is unrelated to the specific target.

The term "designed ankyrin repeat protein" or "DARPin" refers to a non-natural protein comprising an ankyrin repeat domain. In some embodiments, such a DARPin has a repeat sequence motif that was derived from natural ankyrin repeats, e.g. by consensus design (see, e.g., Forrer et al., 2004 Chem Bio Chem, 5, 2, 183-189 and Binz 2003, loc. cit).

The term "fraction of refolded ankyrin repeat domains after thermal denaturation" refers to the fraction of ankyrin repeat domains that refold into their native state after thermal denaturation.

The term "library" as used in reference to a protein or nucleic acid library refers to a collection of proteins and nucleic acids, respectively.

The term "melting temperature" or "Tm" refers to the temperature at which 50% of the protein is unfolded in a certain buffer, e.g. PBS.

The term "modification(s)", as used in reference to a specific amino acid sequence (e.g. the amino acid sequence of an internal ankyrin repeat or capping module), refers to modification(s) of said amino acid sequence selected from the group consisting of deletions, insertions and/or substitutions. In some embodiments, the number of deletions and insertions is limited, for instance, to a combined number of deletions and insertions of not more than three, not more than two or not more than one of the total number of modification(s). Accordingly, if there is a total number of not more than 9 modifications, the number of deletions and insertions of those not more than 9 modifications may be limited to a combined number of insertions and deletions of not more than two. In some embodiments, the modification(s) are substitution(s) only. A substitution can be a substitution of an amino acid residue with, e.g., any of the naturally occurring amino acid residues. In some embodiments, the substitution of an amino acid residue is with an amino acid residue selected from the group consisting of A, D, E, F, H, I, K, L, M, N, Q, R, S, V, Wand Y. In some embodiments, the substitution of an amino acid residue is with an amino acid residue selected from the group consisting of A, D, E, H, I, K, L, Q, R, S, V and Y. The following amino acid residues may, for instance, be particularly suitable for the respective position of the N-terminal capping module:

**Table 1:**

| **Position** | **Amino acid residue** |
|---|---|
| 1 | A, E, N, Q, G, S, T, K, D, R, H, C |
| 2 | E, L, Q, M, K, R, C |
| 3 | G, D, S, A, C |
| 4 | A, R, T, S, N, Q, K, D, E, H, C |
| 5 | A, R, T, S, N, Q, K, D, E, H, C |
| 6 | A, L, N, S, D, C |
| 7 | L, I, V, A, N, T, S, D, C |
| 8 | E, D, Q, A, N, S, T, K, R, H, C |
| 9 | A |
| 10 | V, S, A, C |
| 11 | L, S, Q, K, R, A, H, D, E, C, T, N, F, W, Y |
| 12 | K, R, A, T, S, N, Q, D, E, H, C |
| 13 | G, C |
| 14 | N, S, T, A, D, E, K, Q, R, H, C |
| 15 | M, I, T, A, L, V, S, N, D, Q, K, R, E, C |
| 16 | D, A, N, Q, S, T, K, E, R, H, C |
| 17 | D, A, N, Q, S, T, K, E, R, H, C |
| 18 | T, A, S, I, L, V, C |
| 19 | R, E, D, K, A, N, Q, S, T, H, C |
| 20 | N, K, R, T, S, E, Q, A, D, H, C, I, V |
| 21 | N, S, L, A, C |
| 22 | I, A, V, M, T, L, S, N, C |
| 23 | R, S, Q, K, N, A, E, D, H, C |
| 24 | A, H, K, R, L, I, V, C, G |
| 25 | G |
| 26 | A |
| 27 | N, D, C |
| 28 | T, V, S, P, A, C |
| 29 | D, N, C |
| 30 | A, C |

In some embodiments, the substitution of an amino acid residue is with an amino acid residue selected from the group shown for the respective position in Table 1.

An amino acid substitution may be a conservative or non-conservative substitution. In some embodiments, substitutions only relate to conservative amino acid substitutions. A conservative amino acid substitution typically involves exchanging an amino acid residue by a different one having similar biophysical properties. For instance, the amino acid residues with a positively charged sidechain K, R and H, the amido acids with negatively charged sidechain E and D, the amino acid residue with a polar side chain T and S, the amino acid residues with an aromatic sidechain F, W or Y or the amino acid residues with a non-polar sidechain A, V, L, I and M may be substituted with one another of the respective group.

The term "PBS" refers to phosphate-buffered saline. In some embodiments, PBS contains 137 mM NaCl, 10 mM phosphate and 2.7 mM KCI and has a pH of 7.4.

The term "percent (%) sequence identity" with respect to a specific amino acid sequence (e.g. an amino acid sequence of the N-terminal capping module of the invention) is defined as the percentage of amino acid residues in a candidate sequence that is identical with the amino acid residues in the specific amino acid sequence, after aligning the sequences and introducing gaps, if necessary, to achieve the maximum percent sequence identity, and not considering any conservative substitutions as part of the sequence identity. In some embodiments, such alignment comprises no gaps. Alignment for purposes of determining percent amino acid sequence identity can be achieved in various ways that are within the skill in the art, for instance, using publicly available computer software such as BLAST, BLAST-2 or ALIGN. Those skilled in the art can determine appropriate parameters for measuring alignment, including any algorithms needed to achieve maximal alignment over the full length of the sequences being compared. Further examples of how to determine the percentage of sequence identity can be found in WO 2009/058564 A2, page 93, line 14 to page 102, line 5.

The term "pharmaceutically acceptable carrier" refers to buffers, carriers, and other excipients suitable for use in contact with tissues of humans and/or animals without excessive toxicity, allergic response, irritation, or other problem or complication, commensurate with a reasonable benefit/risk ratio. The carrier(s) should be "acceptable" in the sense of being compatible with the other ingredients of the formulations and not deleterious to the recipient. Pharmaceutically acceptable carriers include buffers, solvents, dispersion media, coatings, isotonic and absorption delaying agents, and the like, that are compatible with pharmaceutical administration.

The term "pharmaceutical composition" refers to a composition comprising at least one active agent and at least one pharmaceutically acceptable carrier. A pharmaceutical composition is generally formulated and administered to exert a pharmaceutically useful effect while minimizing undesirable side effects.

The "position(s)" of the N-terminal capping module referred to herein may relate to the corresponding position(s) of SEQ ID NO: 1, which is the archetypal N-terminal capping module of designed ankyrin repeat proteins that remains commonly used in scientific studies (Binz, 2003, loc. cit.). Accordingly, in some embodiments, the position(s) of the N-terminal capping module relate to the corresponding position(s) of SEQ ID NO: 1. In light of the high sequence similarity of SEQ ID NOs: 1 to 38, the respective positions of these sequences are well aligned and the position(s) of the N-terminal capping module referred to herein may similarly relate to the corresponding position(s) of one or more of SEQ ID NOs: 1 to 38. Accordingly, in some embodiments, the position(s) of the N-terminal capping module relate to the corresponding position(s) of any one of SEQ ID NOs: 1 to 38. In particular, in embodiments further defining the sequence of the N-terminal capping module by way of reference to one or more of SEQ ID NOs: 1 to 38, the position(s) of the N-terminal capping module may relate to the corresponding position(s) of the respective one or more of SEQ ID NOs: 1 to 38 used to further define the sequence of the N-terminal capping module. For instance, for a protein comprising an ankyrin repeat domain having an N-terminal capping module with L at position 24 and comprising a sequence with at least 70% sequence identity to one or more of SEQ ID NOs: 10 to 37, position 24 may refer to the corresponding position of SEQ ID NO: 1, which is N in SEQ ID NO: 1, or it may refer to the corresponding position of any one of SEQ ID NOs: 10 to 37. Furthermore, "corresponding" in this context means that the respective positions align in a sequence alignment. Alignment for purposes of determining which amino acid residue corresponds to which position of a specific sequence can be achieved in various ways that are within the skill in the art, for instance, using publicly available computer software such as BLAST, BLAST-2 or ALIGN. Those skilled in the art can determine appropriate parameters for measuring alignment, including any algorithms needed to achieve maximal alignment over the full length of the sequences being compared. In some embodiment, such alignment comprises no gaps. Further examples of how to align two sequences can be found in WO 2009/058564 A2, page 94, line 7 to page 96, line 28.

The term "recombinant", as used in reference to a protein, refers to a protein produced from a recombinant nucleic acid. A "recombinant nucleic acid" refers to a nucleic acid molecule formed by laboratory methods of genetic recombination or gene synthesis.

The term "substantially identical", as used in reference to a specific amino acid sequence (e.g. the sequence further defining the N-terminal capping module), refers to amino acid sequences having (1) at least 70%, at least 75%, at least 80%, at least 85%, at least 90%, at least 95%, or 100% sequence identity to the specific amino acid sequence or (2) up to 9, up to 8, up to 7, up to 6, up to 5, up to 4, up to 3, up to 2, up to 1 or no modifications, as compared to the specific amino acid sequence.

The term "target", as used, for instance, in conjunction with the specific binding property of an ankyrin repeat domain, refers to any substance or structure. It may refer to a single molecule, such as a protein, peptide, small-molecule or sugar, as well as complexed molecules, such as interacting proteins or proteins binding to non-proteinaceous compounds. It may also refer to more macromolecular structures, such as cells, tissues, viruses or bacteria.

The terms "treating" or "treatment" of a disease, condition or symptom refers to obtaining therapeutic and/or prophylactic benefit, including alleviating, ablating, ameliorating, or preventing a disease, condition or symptom, preventing additional symptoms, ameliorating or preventing the underlying metabolic causes of symptoms, inhibiting the disease or condition, e.g., arresting or slowing down the development of the disease or condition, relieving the disease or condition, causing regression of the disease or condition, relieving a condition caused by the disease or condition, or stopping the symptoms of the disease or condition.

### Detailed description of the invention

Naturally occurring ankyrin repeat domains normally have capping modules to shield their hydrophobic core from the solvent. In line with this, earlier developed recombinant ankyrin repeat domains likewise comprised capping modules with such a shielding function at the N- and C-terminal ends of the ankyrin repeat domain (e.g. Binz, 2003, loc. cit. and Binz, 2004, loc. cit.). The capping modules that were first used were derived from the murine GABPβ1, which is a naturally occurring ankyrin repeat protein (PDB: 1AWC_B). Binz et al. 2003 (loc. cit.) already introduced some modifications to the naturally occurring capping modules in order to adapt the capping modules for binding to the internal ankyrin repeats and for cloning purposes. The N-terminal capping module of Binz et al. 2003 (loc. cit.) is reflected by SEQ ID NO: 1 and the C-terminal capping module of Binz et al. 2003 (loc. cit.) is reflected by SEQ ID NO: 45.

WO 2012/069655 relates to further modified N-terminal and C-terminal capping modules of the ankyrin repeat domain. The N-terminal capping modules disclosed in WO 2012/069655 include the two N-terminal capping modules reflected by SEQ ID NO: 2 and SEQ ID NO: 3 (corresponding to SEQ ID NO: 15 and 14 of WO 2012/069655, respectively, without the two optionally missing N-terminal amino acid residues). Similarly, PCT/EP2021/072819 discloses modified N-terminal capping modules, such as those reflected by SEQ ID Nos: 1 to 76 and 110 of PCT/EP2021/072819.

Using *in silico* structure analysis, the present inventors determined those amino acid residues that would appear to be most suitable for each position of the N-terminal capping module of the ankyrin repeat domain. In particular, the various amino acid residues shown in Table 1 were considered to be particularly suitable based on the *in silico* analysis.

Based on the structural analysis, amino acid residues in various positions were tested by *in vitro* experimentation. Among the many tested mutations of the N-terminal capping module, position 24 was surprisingly found to be particularly important for the thermostability of the ankyrin repeat domain. In particular, it was found that a leucine at this position is particularly favorable.

Accordingly, the protein of the invention comprises an ankyrin repeat domain that has an N-terminal capping module with L at position 24.

In some embodiments, the ankyrin repeat domain of the protein of the invention has improved properties, which may include improved thermostability, improved storage stability, improved thermodynamic stability (defined as the difference in free energy between the folded and unfolded states), improved folding and/or refolding properties (such as a higher fraction of refolded ankyrin repeat domains after thermal denaturation), reduced aggregation propensity and lower *in vivo* immunogenicity risk. Thus, in some embodiments, the protein of the invention comprises an ankyrin repeat domain that has an N-terminal capping module with L at position 24 and an improved thermostability as compared to a reference ankyrin repeat domain having the same sequence except for said position 24, which is a different amino acid residue in the reference ankyrin repeat domain, such as A.

In some embodiments, the N-terminal capping module has further mutations apart from the mutation at position 24.

In some embodiments, the N-terminal capping module further has an amino acid residue of Table 1 in one or more position(s) outside position 24. In some embodiments, the amino acid residue at one or more position(s) outside position 24 of the N-terminal capping module is selected from the group consisting of the amino acid residues shown for the respective position(s) in Table 1.

In some embodiments, the N-terminal capping module further has an amino acid residue selected from the group consisting of L, S, Q, K, R, A, H, D and E at position 11. In some embodiments, the N-terminal capping module further has an amino acid residue selected from the group consisting of K, E, Q, A and L at position 11. In some embodiments, the N-terminal capping module further has an amino acid residue selected from the group consisting of K, E, A and L at position 11. In some embodiments, the N-terminal capping module further has E or A at position 11. In some embodiments, the N-terminal capping module further has A at position 11. In some embodiments, the N-terminal capping module further has E at position 11.

In some embodiments, the N-terminal capping module further has an amino acid residue selected from the group consisting of I, T, A, V, L and M at position 15. In some embodiments, the N-terminal capping module further has I at position 15. In some embodiments, the N-terminal capping module further has V at position 15. In some embodiments, the N-terminal capping module further has L at position 15.

In some embodiments, the N-terminal capping module further has an amino acid residue selected from the group consisting of R, E, D, K, A, N, Q, S, T, H and C at position 19. In some embodiments, the N-terminal capping module further has R at position 19. In some embodiments, the N-terminal capping module further has K at position 19.

In some embodiments, the N-terminal capping module further has L at position 2. In some embodiments, the N-terminal capping module further has L at position 21. In some embodiments, the N-terminal capping module further has L at position 2 and L at position 21.

In some embodiments, the N-terminal capping module further has an amino acid residue selected from the group consisting of L, V, I and A at position 22. In some embodiments, the N-terminal capping module further has an amino acid residue selected from the group consisting of L, V and I at position 22. In some embodiments, the N-terminal capping module further has L at position 22. In some embodiments, the N-terminal capping module further has V at position 22. In some embodiments, the N-terminal capping module further has I at position 22. In some embodiments, the N-terminal capping module further has A at position 22.

In some embodiments, the N-terminal capping module further has an amino acid residue selected from the group consisting of I, T, A, V, L and M at position 15 and an amino acid residue selected from the group consisting of L, V and I at position 22.

In some embodiments, the N-terminal capping module further has L at position 15 and I at position 22. In some embodiments, the N-terminal capping module further has M at position 15 and I at position 22. In some embodiments, the N-terminal capping module further has T at position 15 and I at position 22. In some embodiments, the N-terminal capping module further has I at position 15 and I at position 22. In some embodiments, the N-terminal capping module further has A at position 15 and I at position 22. In some embodiments, the N-terminal capping module further has V at position 15 and I at position 22.

In some embodiments, the N-terminal capping module further has L at position 15 and L at position 22. In some embodiments, the N-terminal capping module further has M at position 15 and L at position 22. In some embodiments, the N-terminal capping module further has T at position 15 and L at position 22. In some embodiments, the N-terminal capping module further has I at position 15 and L at position 22. In some embodiments, the N-terminal capping module further has A at position 15 and L at position 22. In some embodiments, the N-terminal capping module further has V at position 15 and L at position 22.

In some embodiments, the N-terminal capping module further has L at position 15 and V at position 22. In some embodiments, the N-terminal capping module further has M at position 15 and V at position 22. In some embodiments, the N-terminal capping module further has T at position 15 and V at position 22. In some embodiments, the N-terminal capping module further has I at position 15 and V at position 22. In some embodiments, the N-terminal capping module further has A at position 15 and V at position 22. In some embodiments, the N-terminal capping module further has V at position 15 and V at position 22.

In some embodiments, the N-terminal capping module further has an amino acid residue selected from the group consisting of R, S, Q, K, N, A, E, D, H, C at position 23. In some embodiments, the N-terminal capping module further has E at position 23. In some embodiments, the N-terminal capping module further has A at position 23. In some embodiments, the N-terminal capping module further has K at position 23.

In some embodiments, the N-terminal capping module further has an amino acid residue selected from the group consisting of I, T, A, V, L and M at position 15, an amino acid residue selected from the group consisting of R and K at position 19, and an amino acid residue selected from the group consisting of L, V and I at position 22. In some embodiments, the N-terminal capping module further has an amino acid residue selected from the group consisting of I, T, A, V, L and M at position 15, the amino acid residue R at position 19 and an amino acid residue selected from the group consisting of L, V and I at position 22. In some embodiments, the N-terminal capping module further has an amino acid residue selected from the group consisting of I, T, A, V, L and M at position 15, the amino acid residue K at position 19 and an amino acid residue selected from the group consisting of L, V and I at position 22.

In some embodiments, the N-terminal capping module further has an amino acid residue selected from the group consisting of I, T, A, V, L and M at position 15, an amino acid residue selected from the group consisting of L, V and I at position 22, and an amino acid residue selected from the group consisting of A and K at position 23. In some embodiments, the N-terminal capping module further has an amino acid residue selected from the group consisting of I, T, A, V, L and M at position 15, an amino acid residue selected from the group consisting of L, V and I at position 22, and the amino acid residue A at position 23. In some embodiments, the N-terminal capping module further has an amino acid residue selected from the group consisting of I, T, A, V, L and M at position 15, an amino acid residue selected from the group consisting of L, V and I at position 22, and the amino acid residue K at position 23.

In some embodiments, the N-terminal capping module further has an amino acid residue selected from the group consisting of I, T, A, V, L and M at position 15, an amino acid residue selected from the group consisting of R and K at position 19, an amino acid residue selected from the group consisting of L, V and I at position 22, and an amino acid residue selected from the group consisting of A and K at position 23. In some embodiments, the N-terminal capping module further has an amino acid residue selected from the group consisting of I, T, A, V, L and M at position 15, the amino acid residue R at position 19, an amino acid residue selected from the group consisting of L, V and I at position 22, and the amino acid residue K at position 23. In some embodiments, the N-terminal capping module further has an amino acid residue selected from the group consisting of I, T, A, V, L and M at position 15, the amino acid residue K at position 19, an amino acid residue selected from the group consisting of L, V and at position 22 I, and the amino acid residue K at position 23. In some embodiments, the N-terminal capping module further has an amino acid residue selected from the group consisting of I, T, A, V, L and M at position 15, the amino acid residue R at position 19, an amino acid residue selected from the group consisting of L, V and I at position 22, and the amino acid residue A at position 23. In some embodiments, the N-terminal capping module further has an amino acid residue selected from the group consisting of I, T, A, V, L and M at position 15, the amino acid residue K at position 19, an amino acid residue selected from the group consisting of L, V and I at position 22, and the amino acid residue A at position 23.

In some embodiments, the N-terminal capping module further has an amino acid residue selected from the group consisting of E and A at position 11, an amino acid residue selected from the group consisting of I, T, A, V, L and M at position 15, an amino acid residue selected from the group consisting of R and K at position 19, an amino acid residue selected from the group consisting of L, V and I at position 22, and an amino acid residue selected from the group consisting of A and K at position 23.

In some embodiments, the N-terminal capping module further has an amino acid residue selected from the group consisting of R and K at position 19 and an amino acid residue selected from the group consisting of A and K at position 23. In some embodiments, the N-terminal capping module further has R at position 19 and A at position 23. In some embodiments, the N-terminal capping module further has K at position 19 and A at position 23. In some embodiments, the N-terminal capping module further has R at position 19 and K at position 23. In some embodiments, the N-terminal capping module further has K at position 19 and K at position 23.

In some embodiments, the N-terminal capping module comprises the amino acid sequence (R/K)(I/E)L(L/I/M)(A/K)L at positions 19 to 24, wherein the amino acid residue at the positions 19, 20, 22 and 23 is selected from the group consisting of the amino acid residues shown in the respective parentheses. In some embodiments, the N-terminal capping module comprises one of the amino acid residues indicated for the respective positions in Table 1 at positions 19 to 23, and L at position 24. In some embodiments, the N-terminal capping module comprises an amino acid sequence at positions 19 to 24 selected from the group consisting of: RILMAL, RELLKL, RILLKL, RELIKL, RILIKL, RELLAL, RILLAL, RELIAL, RILIAL, KILMAL, KELLKL, KILLKL, KELIKL, KILIKL, KELLAL, KILLAL, KELIAL and KILIAL.

In some embodiments, the N-terminal capping module comprises an amino acid residue selected from the group consisting of I, T, A, V, L and M at position 15, and (R/K)(I/E)L(L/I/M)(A/K)L at positions 19 to 24, wherein the amino acid residue at the positions 19, 20, 22 and 23 is selected from the group consisting of the amino acid residues shown in the respective parentheses. In some embodiments, the N-terminal capping module comprises an amino acid residue selected from the group consisting of I, T, A, V, L and M at position 15, one of the amino acid residues indicated for the respective positions in Table 1 at positions 19 to 23, and L at position 24. In some embodiments, the N-terminal capping module comprises an amino acid residue selected from the group consisting of I, T, A, V, L and M at position 15, and an amino acid sequence at positions 19 to 24 selected from the group consisting of: RILMAL, RELLKL, RILLKL, RELIKL, RILIKL, RELLAL, RILLAL, RELIAL, RILIAL, KILMAL, KELLKL, KILLKL, KELIKL, KILIKL, KELLAL, KILLAL, KELIAL and KILIAL.

In some embodiments, the ankyrin repeat domain of the protein of the invention has an improved thermostability, such as a higher melting temperature and/or a higher fraction of refolded ankyrin repeat domains after thermal denaturation, as compared to a reference ankyrin repeat domain having the same amino acid sequence except for the mutation at position 24 of the N-terminal capping module. In some embodiments having one or more of said mutation(s) in the N-terminal capping module in addition to the mutation at position 24, the ankyrin repeat domain of the protein of the invention has an improved thermostability, such as a higher melting temperature and/or a higher fraction of refolded ankyrin repeat domains after thermal denaturation, as compared to a reference ankyrin repeat domain having the same amino acid sequence except for the mutation at position 24 of the N-terminal capping module and except for the additional mutation(s) in the N-terminal capping module. In some embodiments having one or more of said mutation(s) in the N-terminal capping module in addition to the mutation at position 24, the ankyrin repeat domain of the protein of the invention has an improved thermostability, such as a higher melting temperature and/or a higher fraction of refolded ankyrin repeat domains after thermal denaturation, as compared to a reference ankyrin repeat domain having the same amino acid sequence except for the mutation at position 24 of the N-terminal capping module alone. The respective amino acid residue(s) that are different in the reference ankyrin repeat domain may, for instance, refer to the amino acid residue(s) found in the corresponding position(s) of SEQ ID NO: 1 or SEQ ID NO: 2. In some embodiments, the amino acid residue at position 24 of the reference ankyrin repeat domain is selected from the naturally occurring amino acid residues other than L. In some embodiments, the amino acid residue at position 24 of the reference ankyrin repeat domain is selected from the amino acid residues shown in Table 1 for position 24 other than L. In some embodiments, the amino acid residue at position 24 of the reference ankyrin repeat domain is N or A. In some embodiments, the amino acid residue at position 24 of the reference ankyrin repeat domain is A. In some embodiments additionally having position 15 of the N-terminal capping module mutated, the amino acid residue at position 15 of the reference ankyrin repeat domain is D and the amino acid residue at position 24 of the reference ankyrin repeat domain is A. In some embodiments additionally having positions 15 and 22 of the N-terminal capping module mutated, the amino acid residue at position 15 of the reference ankyrin repeat domain is D, the amino acid residue at position 22 of the reference ankyrin repeat domain is M and the amino acid residue at position 24 of the reference ankyrin repeat domain is A.

Methods for measuring the thermostability of a protein or a protein domain are well-known to the person skilled in the art. For instance, the thermostability can be measured by a thermal shift assay, differential scanning calorimetry and circular dichroism (CD). Another possible approach is to use differential scanning fluorimetry (e.g. Nielsen et al., 2007, Nat Protoc. 2, 9:2212-21). In this method, unfolding of the protein is measured with a fluorescent dye that binds to hydrophobic parts of the protein. As the protein unfolds, more hydrophobic parts become exposed causing an increase in fluorescence and vice versa. This method therefore allows to conveniently monitor the refolding properties of a protein and to determine its melting temperature, which corresponds to the midpoint of the fluorescence transition curve. The refolding properties and melting temperature of a protein can also be measured by CD spectroscopy, whereby the thermal melting curve of the protein is determined by measuring the CD signal at 222 nm. For purposes of measuring the thermostability, the protein may be dissolved in PBS. For example, the thermostability of a helical protein, such as an ankyrin repeat domain, can be determined by measuring the CD signal of the protein at 222 nm while slowly heating the protein at a concentration of 0.01 mM in PBS pH 7.4 from 20°C to 95°C using a temperature ramp of 1°C per min. A denaturant, such as guanidine chloride, may be added to the PBS buffer, e.g., if measuring a protein that does not fully unfold at 95°C.

In some embodiments, the increase in melting temperature of the ankyrin repeat domain of the invention is at least 1°C, at least 2°C, at least 3°C, at least 4°C or at least 5°C, as compared to the reference ankyrin repeat domain.

In some embodiments, the fraction of the refolded ankyrin repeat domains after thermal denaturation is at least 1%, at least 5%, at least 10% or at least 20% higher, as compared to the reference ankyrin repeat domain.

Unless specified, the sequence of the ankyrin repeat domain is not particularly limited. In particular, the ankyrin repeat domain allows for a large sequence variation while preserving the overall structure and function of the domain.

In some embodiments, the N-terminal capping module comprises an amino acid sequence that is substantially identical to an amino acid sequence selected from the group consisting of SEQ ID NOs: 1 to 38. In some embodiments, the N-terminal capping module comprises an amino acid sequence that is substantially identical to an amino acid sequence selected from the group consisting of SEQ ID NOs: 1 to 37. In some embodiments, the N-terminal capping module comprises an amino acid sequence that is substantially identical to an amino acid sequence selected from the group consisting of SEQ ID NOs: 10 to 37. In some embodiments, the N-terminal capping module comprises an amino acid sequence that is substantially identical to an amino acid sequence selected from the group consisting of SEQ ID NOs: 20 to 37. In some embodiments, the N-terminal capping module comprises an amino acid sequence that is substantially identical to SEQ ID NO: 38. In some embodiments, the N-terminal capping module comprises a sequence that is substantially identical to positions 1 to 30 of the N-terminal capping module of any one of the ankyrin repeat domains used in the present examples (positions 1 to 30 of the N-terminal capping module correspond to positions 13 to 42 of SEQ ID NOs: 58 to 97).

In some embodiments, the N-terminal capping module comprises an amino acid sequence that has at least 70%, at least 75%, at least 80%, at least 85%, at least 90%, at least 95%, or 100% sequence identity to an amino acid sequence selected from the group consisting of SEQ ID NOs: 1 to 38. In some embodiments, the N-terminal capping module comprises an amino acid sequence that has at least 70%, at least 75%, at least 80%, at least 85%, at least 90%, at least 95%, or 100% sequence identity to an amino acid sequence selected from the group consisting of SEQ ID NOs: 1 to 37. In some embodiments, the N-terminal capping module comprises an amino acid sequence that has at least 70%, at least 75%, at least 80%, at least 85%, at least 90%, at least 95%, or 100% sequence identity to an amino acid sequence selected from the group consisting of SEQ ID NOs: 10 to 37. In some embodiments, the N-terminal capping module comprises an amino acid sequence that has at least 70%, at least 75%, at least 80%, at least 85%, at least 90%, at least 95%, or 100% sequence identity to an amino acid sequence selected from the group consisting of SEQ ID NOs: 20 to 37. In some embodiments, the N-terminal capping module comprises an amino acid sequence that has at least 70%, at least 75%, at least 80%, at least 85%, at least 90%, at least 95%, or 100% sequence identity to SEQ ID NO: 38.

In some embodiments, the N-terminal capping module comprises an amino acid sequence selected from any one of SEQ ID NOs: 1 to 38 or from a variant of any one of SEQ ID NOs: 1 to 38 with up to 9, up to 8, up to 7, up to 6, up to 5, up to 4, up to 3, up to 2 or up to 1 modification(s). In some embodiments, the N-terminal capping module comprises an amino acid sequence selected from any one of SEQ ID NOs: 1 to 37 or from a variant of any one of SEQ ID NOs: 1 to 37 with up to 9, up to 8, up to 7, up to 6, up to 5, up to 4, up to 3, up to 2 or up to 1 modification(s). In some embodiments, the N-terminal capping module comprises an amino acid sequence selected from any one of SEQ ID NOs: 10 to 37 or from a variant of any one of SEQ ID NOs: 10 to 37 with up to 9, up to 8, up to 7, up to 6, up to 5, up to 4, up to 3, up to 2 or up to 1 modification(s). In some embodiments, the N-terminal capping module comprises an amino acid sequence selected from any one of SEQ ID NOs: 20 to 37 or from a variant of any one of SEQ ID NOs: 20 to 37 with up to 9, up to 8, up to 7, up to 6, up to 5, up to 4, up to 3, up to 2 or up to 1 modification(s). In some embodiments, the N-terminal capping module comprises the amino acid sequence of SEQ ID NO: 38 or the amino acid sequence of a variant of SEQ ID NO: 38 with up to 9, up to 8, up to 7, up to 6, up to 5, up to 4, up to 3, up to 2 or up to 1 modification(s). In some embodiments, there are no variants of the above sequences in this paragraph. In some embodiments, the modifications of the above sequences in this paragraph do not include more than a combined number of deletions and insertions of 3. In some embodiments, the modifications of the above sequences in this paragraph do not include more than a combined number of deletions and insertions of 2. In some embodiments, the modifications of the above sequences in this paragraph do not include more than a combined number of deletions and insertions of 1. In some embodiments, the modifications of the above sequences in this paragraph are only substitutions. In some embodiments, the variants of the above sequences in this paragraph comprise one or more substitutions with an amino acid residue of Table 1. In some embodiments, the variants of the above sequences in this paragraph only comprise substitutions with amino acid residues of Table 1.

The N-terminal capping module may further comprise a sequence directly N-terminal to the amino acid sequences defined in SEQ ID NOs: 1 to 38 (or the sequence variants thereof defined herein). For instance, such sequence could be a dipeptide comprising amino acid residues selected from the group consisting of D, A, E, N, Q, S, T, K, R and H, such as the dipeptide GS, DA, EA, AA, AD, AE, NA, AN, PT, TP, AT or TA. For instance, G and S or D and A could be at positions -2 and -1 of the N-terminal capping module, respectively. Such dipeptide sequence may serve as a linker to connect the ankyrin repeat domain to the further peptide sequence of the protein or as an extended alpha-helix of the N-terminal capping module.

It is understood that for those embodiments of the N-terminal capping module, which are defined by a certain amino acid residue(s) in a position(s), e.g., L at position 24, as well as a minimal sequence identity to an amino acid sequence or a defined number of modifications compared to an amino acid sequence, both conditions need to be fulfilled. For instance, an N-terminal capping module having L at position 24 and at least 70% sequence identity to SEQ ID NOs: 1 to 38, only relates to such embodiments wherein the N-terminal capping module has L at position 24 and, at the same time, at least 70% sequence identity to one or more of SEQ ID NOs: 1 to 38. Similarly, an N-terminal capping module having L at position 24 and comprising the amino acid sequence of any one of SEQ ID NOs: 1 to 38 or a variant of any one of SEQ ID NOs: 1 to 38 with up to 9 modifications, only relates to such embodiments wherein the N-terminal capping module has L at position 24 and, at the same time, comprises a sequence corresponding to any one of SEQ ID NOs: 1 to 38 or said variants thereof.

In some embodiments, the internal ankyrin repeat(s) of the ankyrin repeat domain consist of 33 amino acid residues.

In some embodiments, at least one internal ankyrin repeat of the ankyrin repeat domain of the invention comprises an amino acid sequence that is substantially identical to an amino acid sequence selected from the group consisting of SEQ ID NOs: 39 to 44 or from the group consisting of SEQ ID NOs: 40 to 44. In some embodiments, at least one internal ankyrin repeat comprises an amino acid sequence that has at least 70%, at least 75%, at least 80%, at least 85%, at least 90%, at least 95%, or 100% sequence identity to an amino acid sequence selected from the group consisting of SEQ ID NOs: 39 to 44. In some embodiments, at least one internal ankyrin repeat comprises an amino acid sequence selected from the group consisting of SEQ ID NOs: 39 to 44 or from a variant of any one of SEQ ID NOs: 39 to 44 with up to 9, up to 8, up to 7, up to 6, up to 5, up to 4, up to 3, up to 2 or up to 1 modification(s).

In some embodiments, each internal ankyrin repeat (of the one or more internal ankyrin repeats) of the ankyrin repeat domain comprises an amino acid sequence as defined above for the at least one internal ankyrin repeat of the ankyrin repeat domain of the invention. Accordingly, if the ankyrin repeat domain has only one internal ankyrin repeat, this internal ankyrin repeat has an amino acid sequence as defined above. Similarly, if the ankyrin repeat domain has two internal ankyrin repeats, both internal ankyrin repeats have an amino acid sequence as defined above and so on. In some embodiments, each internal ankyrin repeat of the ankyrin repeat domain of the invention comprises an amino acid sequence that is substantially identical to an amino acid sequence selected from the group consisting of SEQ ID NOs: 39 to 44 or from the group consisting of SEQ ID NOs: 40 to 44. In some embodiments, each internal ankyrin repeat comprises an amino acid sequence that has at least 70%, at least 75%, at least 80%, at least 85%, at least 90%, at least 95%, or 100% sequence identity to an amino acid sequence selected from the group consisting of SEQ ID NOs: 39 to 44. In some embodiments, each internal ankyrin repeat comprises an amino acid sequence selected from the group consisting of SEQ ID NOs: 39 to 44 or from a variant of any one of SEQ ID NOs: 39 to 44 with up to 9, up to 8, up to 7, up to 6, up to 5, up to 4, up to 3, up to 2 or up to 1 modification(s).

In some embodiments, the internal ankyrin repeat adjacent to the N-terminal capping module of the ankyrin repeat domain comprises an amino acid sequence as defined above for the at least one internal ankyrin repeat of the ankyrin repeat domain of the invention. In some embodiments, the internal ankyrin repeat adjacent to the N-terminal capping module of the ankyrin repeat domain of the invention comprises an amino acid sequence that is substantially identical to an amino acid sequence selected from the group consisting of SEQ ID NOs: 39 to 44 or from the group consisting of SEQ ID NOs: 40 to 44. In some embodiments, the internal ankyrin repeat adjacent to the N-terminal capping module comprises an amino acid sequence that has at least 70%, at least 75%, at least 80%, at least 85%, at least 90%, at least 95%, or 100% sequence identity to an amino acid sequence selected from the group consisting of SEQ ID NOs: 39 to 44. In some embodiments, the internal ankyrin repeat adjacent to the N-terminal capping module comprises an amino acid sequence selected from the group consisting of SEQ ID NOs: 39 to 44 or from a variant of any one of SEQ ID NOs: 39 to 44 with up to 9, up to 8, up to 7, up to 6, up to 5, up to 4, up to 3, up to 2 or up to 1 modification(s).

In some embodiments, the internal ankyrin repeats of the ankyrin repeat domain of the invention share a high degree of sequence identity. In some embodiments, the internal ankyrin repeat(s) share at least 70%, at least 75%, at least 80%, at least 85%, at least 90% or at least 95% sequence identity.

In some embodiments, the C-terminal capping module of the ankyrin repeat domain of the invention comprises an amino acid sequence that is substantially identical to an amino acid sequence selected from the group consisting of SEQ ID NOs: 45 to 57. In some embodiments, the C-terminal capping module comprises an amino acid sequence that has at least 70%, at least 75%, at least 80%, at least 85%, at least 90%, at least 95%, or 100% sequence identity to an amino acid sequence selected from the group consisting of SEQ ID NOs: 45 to 57. In some embodiments, the C-terminal capping module comprises an amino acid sequence selected from the group consisting of SEQ ID NOs: 45 to 57 or from a variant of any one of SEQ ID NOs: 45 to 57 with up to 9, up to 8, up to 7, up to 6, up to 5, up to 4, up to 3, up to 2 or up to 1 modification(s).

In some embodiments, the N-terminal capping module of the ankyrin repeat domain of the invention comprises an amino acid sequence that is substantially identical to an amino acid sequence selected from the group consisting of SEQ ID NOs: 1 to 38, such as an amino acid sequence selected from the group consisting of SEQ ID NOs: 1 to 37, and each internal ankyrin repeat comprises an amino acid sequence that is substantially identical to an amino acid sequence selected from the group consisting of SEQ ID NOs: 39 to 44. In some embodiments, the N-terminal capping module of the ankyrin repeat domain of the invention comprises an amino acid sequence that is substantially identical to an amino acid sequence selected from the group consisting of SEQ ID NOs: 1 to 38, such as an amino acid sequence selected from the group consisting of SEQ ID NOs: 1 to 37, and at least one internal ankyrin repeat comprises an amino acid sequence that is substantially identical to an amino acid sequence selected from the group consisting of SEQ ID NOs: 39 to 44.

In some embodiments, the N-terminal capping module comprises an amino acid sequence that has at least 70%, at least 75%, at least 80%, at least 85%, at least 90%, at least 95%, or 100% sequence identity to an amino acid sequence selected from the group consisting of SEQ ID NOs: 1 to 38, such as an amino acid sequence selected from the group consisting of SEQ ID NOs: 1 to 37, and each internal ankyrin repeat comprises an amino acid sequence that has at least 70%, at least 75%, at least 80%, at least 85%, at least 90%, at least 95%, or 100% sequence identity to an amino acid sequence selected from the group consisting of SEQ ID NOs: 39 to 44. In some embodiments, the N-terminal capping module comprises an amino acid sequence that has at least 70%, at least 75%, at least 80%, at least 85%, at least 90%, at least 95%, or 100% sequence identity to an amino acid sequence selected from the group consisting of SEQ ID NOs: 1 to 38, such as an amino acid sequence selected from the group consisting of SEQ ID NOs: 1 to 37, and at least one internal ankyrin repeat comprises an amino acid sequence that has at least 70%, at least 75%, at least 80%, at least 85%, at least 90%, at least 95%, or 100% sequence identity to an amino acid sequence selected from the group consisting of SEQ ID NOs: 39 to 44. In some embodiments, the sequence identity to the above sequences of the N-terminal capping module and internal ankyrin repeat in this paragraph is at least 70%. In some embodiments, the sequence identity to the above sequences of the N-terminal capping module and internal ankyrin repeat in this paragraph is at least 75%. In some embodiments, the sequence identity to the above sequences of the N-terminal capping module and internal ankyrin repeat in this paragraph is at least 80%. In some embodiments, the sequence identity to the above sequences of the N-terminal capping module and internal ankyrin repeat in this paragraph is at least 85%. In some embodiments, the sequence identity to the above sequences of the N-terminal capping module and internal ankyrin repeat in this paragraph is at least 90%. In some embodiments, the sequence identity to the above sequences of the N-terminal capping module and internal ankyrin repeat in this paragraph is at least 95%. In some embodiments, the sequence identity to the above sequences of the N-terminal capping module and internal ankyrin repeat in this paragraph is 100%.

In some embodiments, the N-terminal capping module comprises an amino acid sequence selected from the group consisting of SEQ ID NOs: 1 to 38 or from a variant of any one of SEQ ID NOs: 1 to 38 with up to 9, up to 8, up to 7, up to 6, up to 5, up to 4, up to 3, up to 2 or up to 1 modification(s) and each internal ankyrin repeat comprises an amino acid sequence selected from the group consisting of SEQ ID NOs: 39 to 44 or from a variant of any one of SEQ ID NOs: 39 to 44 with up to 9, up to 8, up to 7, up to 6, up to 5, up to 4, up to 3, up to 2 or up to 1 modification(s). In some embodiments, the N-terminal capping module comprises an amino acid sequence selected from the group consisting of SEQ ID NOs: 1 to 38 or from a variant of any one of SEQ ID NOs: 1 to 38 with up to 9, up to 8, up to 7, up to 6, up to 5, up to 4, up to 3, up to 2 or up to 1 modification(s) and at least one internal ankyrin repeat comprises an amino acid sequence selected from the group consisting of SEQ ID NOs: 39 to 44 or from a variant of any one of SEQ ID NOs: 39 to 44 with up to 9, up to 8, up to 7, up to 6, up to 5, up to 4, up to 3, up to 2 or up to 1 modification(s). In some embodiments, the variants of the above sequences of the N-terminal capping module and internal ankyrin repeat in this paragraph have up to 9 modifications. In some embodiments, the variants of the above sequences of the N-terminal capping module and internal ankyrin repeat in this paragraph have up to 8 modifications. In some embodiments, the variants of the above sequences of the N-terminal capping module and internal ankyrin repeat in this paragraph have up to 7 modifications. In some embodiments, the variants of the above sequences of the N-terminal capping module and internal ankyrin repeat in this paragraph have up to 6 modifications. In some embodiments, the variants of the above sequences of the N-terminal capping module and internal ankyrin repeat in this paragraph have up to 5 modifications. In some embodiments, the variants of the above sequences of the N-terminal capping module and internal ankyrin repeat in this paragraph have up to 4 modifications. In some embodiments, the variants of the above sequences of the N-terminal capping module and internal ankyrin repeat in this paragraph have up to 3 modifications. In some embodiments, the variants of the above sequences of the N-terminal capping module and internal ankyrin repeat in this paragraph have up to 2 modifications. In some embodiments, the variants of the above sequences of the N-terminal capping module and internal ankyrin repeat in this paragraph have up to 1 modification. In some embodiments, there are no variants of the above sequences of the N-terminal capping module and internal ankyrin repeat in this paragraph. In some embodiments, the modifications of the above sequences of the N-terminal capping module and internal ankyrin repeat in this paragraph do not include more than a combined number of deletions and insertions of 3. In some embodiments, the modifications of the above sequences of the N-terminal capping module and internal ankyrin repeat in this paragraph do not include more than a combined number of deletions and insertions of 2. In some embodiments, the modifications of the above sequences of the N-terminal capping module and internal ankyrin repeat in this paragraph do not include more than a combined number of deletions and insertions of 1. In some embodiments, the modifications of the above sequences of the N-terminal capping module and internal ankyrin repeat in this paragraph are only substitutions.

In some embodiments, the N-terminal capping module comprises an amino acid sequence that is substantially identical to an amino acid sequence selected from the group consisting of SEQ ID NOs: 1 to 38, such as an amino acid sequence selected from the group consisting of SEQ ID NOs: 1 to 37, each internal ankyrin repeat comprises an amino acid sequence that is substantially identical to an amino acid sequence selected from the group consisting of SEQ ID NOs: 39 to 44, and the C-terminal capping module comprises an amino acid sequence that is substantially identical to an amino acid sequence selected from the group consisting of SEQ ID NOs: 45 to 57. In some embodiments, the N-terminal capping module comprises an amino acid sequence that is substantially identical to an amino acid sequence selected from the group consisting of SEQ ID NOs: 1 to 38, such as an amino acid sequence selected from the group consisting of SEQ ID NOs: 1 to 37, at least one internal ankyrin repeat comprises an amino acid sequence that is substantially identical to an amino acid sequence selected from the group consisting of SEQ ID NOs: 39 to 44, and the C-terminal capping module comprises an amino acid sequence that is substantially identical to an amino acid sequence selected from the group consisting of SEQ ID NOs: 45 to 57.

In some embodiments, the N-terminal capping module comprises an amino acid sequence that has at least 70%, at least 75%, at least 80%, at least 85%, at least 90%, at least 95%, or 100% sequence identity to an amino acid sequence selected from the group consisting of SEQ ID NOs: 1 to 38, such as an amino acid sequence selected from the group consisting of SEQ ID NOs: 1 to 37, each internal ankyrin repeat comprises an amino acid sequence that has at least 70%, at least 75%, at least 80%, at least 85%, at least 90%, at least 95%, or 100% sequence identity to an amino acid sequence selected from the group consisting of SEQ ID NOs: 39 to 44, and the C-terminal capping module comprises an amino acid sequence that has at least 70%, at least 75%, at least 80%, at least 85%, at least 90%, at least 95%, or 100% sequence identity to an amino acid sequence selected from the group consisting of SEQ ID NOs: 45 to 57. In some embodiments, the N-terminal capping module comprises an amino acid sequence that has at least 70%, at least 75%, at least 80%, at least 85%, at least 90%, at least 95%, or 100% sequence identity to an amino acid sequence selected from the group consisting of SEQ ID NOs: 1 to 38, such as an amino acid sequence selected from the group consisting of SEQ ID NOs: 1 to 37, at least one internal ankyrin repeat comprises an amino acid sequence that has at least 70%, at least 75%, at least 80%, at least 85%, at least 90%, at least 95%, or 100% sequence identity to an amino acid sequence selected from the group consisting of SEQ ID NOs: 39 to 44, and the C-terminal capping module comprises an amino acid sequence that has at least 70%, at least 75%, at least 80%, at least 85%, at least 90%, at least 95%, or 100% sequence identity to an amino acid sequence selected from the group consisting of SEQ ID NOs: 45 to 57. In some embodiments, the sequence identity to the above sequences of the N-terminal capping module, internal ankyrin repeat and C-terminal capping module in this paragraph is at least 70%. In some embodiments, the sequence identity to the above sequences of the N-terminal capping module, internal ankyrin repeat and C-terminal capping module in this paragraph is at least 75%. In some embodiments, the sequence identity to the above sequences of the N-terminal capping module, internal ankyrin repeat and C-terminal capping module in this paragraph is at least 80%. In some embodiments, the sequence identity to the above sequences of the N-terminal capping module, internal ankyrin repeat and C-terminal capping module in this paragraph is at least 85%. In some embodiments, the sequence identity to the above sequences of the N-terminal capping module, internal ankyrin repeat and C-terminal capping module in this paragraph is at least 90%. In some embodiments, the sequence identity to the above sequences of the N-terminal capping module, internal ankyrin repeat and C-terminal capping module in this paragraph is at least 95%. In some embodiments, the sequence identity to the above sequences of the N-terminal capping module, internal ankyrin repeat and C-terminal capping module in this paragraph is 100%.

In some embodiments, the N-terminal capping module comprises an amino acid sequence selected from the group consisting of SEQ ID NOs: 1 to 38 or from a variant of any one of SEQ ID NOs: 1 to 38 with up to 9, up to 8, up to 7, up to 6, up to 5, up to 4, up to 3, up to 2 or up to 1 modification(s), each internal ankyrin repeat comprises an amino acid sequence selected from the group consisting of SEQ ID NOs: 39 to 44 or from a variant of any one of SEQ ID NOs: 39 to 44 with up to 9, up to 8, up to 7, up to 6, up to 5, up to 4, up to 3, up to 2 or up to 1 modification(s), and the C-terminal capping module comprises an amino acid sequence selected from the group consisting of SEQ ID NOs: 45 to 57 or from a variant of any one of SEQ ID NOs: 45 to 57 with up to 9, up to 8, up to 7, up to 6, up to 5, up to 4, up to 3, up to 2 or up to 1 modification(s). In some embodiments, the N-terminal capping module comprises an amino acid sequence selected from the group consisting of SEQ ID NOs: 1 to 38 or from a variant of any one of SEQ ID NOs: 1 to 38 with up to 9, up to 8, up to 7, up to 6, up to 5, up to 4, up to 3, up to 2 or up to 1 modification(s), at least one internal ankyrin repeat comprises an amino acid sequence selected from the group consisting of SEQ ID NOs: 39 to 44 or from a variant of any one of SEQ ID NOs: 39 to 44 with up to 9, up to 8, up to 7, up to 6, up to 5, up to 4, up to 3, up to 2 or up to 1 modification(s), and the C-terminal capping module comprises an amino acid sequence selected from the group consisting of SEQ ID NOs: 45 to 57 or from a variant of any one of SEQ ID NOs: 45 to 57 with up to 9, up to 8, up to 7, up to 6, up to 5, up to 4, up to 3, up to 2 or up to 1 modification(s). In some embodiments, the variants of the above sequences of the N-terminal capping module, internal ankyrin repeat and C-terminal capping module in this paragraph have up to 9 modifications. In some embodiments, the variants of the above sequences of the N-terminal capping module, internal ankyrin repeat and C-terminal capping module in this paragraph have up to 8 modifications. In some embodiments, the variants of the above sequences of the N-terminal capping module, internal ankyrin repeat and C-terminal capping module in this paragraph have up to 7 modifications. In some embodiments, the variants of the above sequences of the N-terminal capping module, internal ankyrin repeat and C-terminal capping module in this paragraph have up to 6 modifications. In some embodiments, the variants of the above sequences of the N-terminal capping module, internal ankyrin repeat and C-terminal capping module in this paragraph have up to 5 modifications. In some embodiments, the variants of the above sequences of the N-terminal capping module, internal ankyrin repeat and C-terminal capping module in this paragraph have up to 4 modifications. In some embodiments, the variants of the above sequences of the N-terminal capping module, internal ankyrin repeat and C-terminal capping module in this paragraph have up to 3 modifications. In some embodiments, the variants of the above sequences of the N-terminal capping module, internal ankyrin repeat and C-terminal capping module in this paragraph have up to 2 modifications. In some embodiments, the variants of the above sequences of the N-terminal capping module, internal ankyrin repeat and C-terminal capping module in this paragraph have up to 1 modification. In some embodiments, there are no variants of the above sequences of the N-terminal capping module, internal ankyrin repeat and C-terminal capping module in this paragraph. In some embodiments, the modifications of the above sequences of the N-terminal capping module, internal ankyrin repeat and C-terminal capping module in this paragraph do not include more than a combined number of deletions and insertions of 3. In some embodiments, the modifications of the above sequences of the N-terminal capping module, internal ankyrin repeat and C-terminal capping module in this paragraph do not include more than a combined number of deletions and insertions of 2. In some embodiments, the modifications of the above sequences of the N-terminal capping module, internal ankyrin repeat and C-terminal capping module in this paragraph do not include more than a combined number of deletions and insertions of 1. In some embodiments, the modifications of the above sequences of the N-terminal capping module, internal ankyrin repeat and C-terminal capping module in this paragraph are only substitutions.

In some embodiments, the ankyrin repeat domain comprises the N-terminal capping module, one internal ankyrin repeat and a C-terminal capping module (such ankyrin repeat domain structure is also referred to as "N1C"). Such ankyrin repeat domains are shown in Example 1. In some embodiments, the ankyrin repeat domain comprises the N-terminal capping module, multiple internal ankyrin repeats, such as 2, 3, 4 or 5 internal ankyrin repeats, and a C-terminal capping module. In one embodiment, the ankyrin repeat domain comprises the N-terminal capping module, 2 or 3 internal ankyrin repeats and a C-terminal capping module (such ankyrin repeat domain structure is also referred to as "N2C" or "N3C", respectively). In one embodiment, the ankyrin repeat domain has a N2C structure. In another embodiment, the ankyrin repeat domain has a N3C structure.

In some embodiments, the protein of the invention is a recombinant protein or a DARPin.

In some embodiments, the ankyrin repeat domain of the protein of the invention specifically binds to a target. For instance, the ankyrin repeat domain may specifically bind to a mammalian serum albumin, such as human serum albumin. Exemplary ankyrin repeat domains specifically binding to human serum albumin are disclosed in WO 2012/069654 A1 and also found in ensovibep (see amino acid residues 1-126 and 149-274 of ensovibep, respectively, as defined, e.g., in Proposed INN List: 124; WHO Drug Information, Vol. 34, No. 4, 2020). In some embodiments, the target is a peptide-MHC complex. In some embodiments, the target is a protein on a cell surface, such as Her2, CD3, CD4, CD8, CD33, CD40, CD70, CD123, FAP or 4-1BB. In some embodiments, the target is an intracellular protein. In some embodiments, the target is a protein on the surface of a virus. In some embodiments, the target is a blood-circulating protein, such as VEGF.

In some embodiments, the protein only comprises a single ankyrin repeat domain.

The protein may also comprise one or more further moieties in addition to the ankyrin repeat domain having the N-terminal capping module of the invention, such as a moiety binding to a target, a labeling moiety, a toxic moiety, a moiety improving the pharmacokinetics, a moiety providing effector functions, a moiety allowing for the purification of the protein or a moiety providing enzymatic activity. In some embodiments, the further moiety binding to a target is another ankyrin repeat domain, an antibody or fragment thereof or a receptor protein. In some embodiments, the further moiety binding to a target is another ankyrin repeat domain. In some embodiments, the labeling moiety is a stable isotope, a mass tag or a fluorescent label. In some embodiments, the toxic moiety is a chemotherapeutic agent, such as an alkylating agent, an antimetabolite, a taxane, or an anthracycline. In some embodiments, the moiety improving pharmacokinetics is a polypeptide (e.g., as used for PASylation), polyethylene glycol (PEG), a mammalian serum albumin, an immunoglobulin, a Fc domain of an immunoglobulin or a moiety binding to mammalian serum albumin or to an immunoglobulin. In one embodiment, the protein further contains an ankyrin repeat domain binding to a mammalian serum albumin. In some embodiments, the further moiety providing effector functions is a Fc domain of an immunoglobulin. In some embodiments, the moiety allowing for the purification of the protein is a FLAG-tag, a GST-tag, an HA-tag, a Myc-tag, a His-tag or a Strep-tag. In some embodiments, the further moiety providing enzymatic or fluorescence activity is, e.g., beta-lactamase or green fluorescence protein, respectively.

The further moiety may be proteinaceous or non-proteinaceous.

In some embodiments, the further moiety in addition to the ankyrin repeat domain having the N-terminal capping module of the invention is one or more additional ankyrin repeat domain(s). In some embodiments, one or more of the additional ankyrin repeat domain(s) is an ankyrin repeat domain of the invention and thus has L at position 24 of the N-terminal capping module. In some embodiments, none of the additional one or more ankyrin repeat domain(s) has L at position 24 of the N-terminal capping module. In some embodiments, all of the additional ankyrin repeat domain(s) are ankyrin repeat domains of the invention having L at position 24 of the N-terminal capping module. In some embodiments, all ankyrin repeat domains have the same N-terminal capping module. In some embodiments, the protein of the invention comprises more than one, e.g., at least two, at least three, at least four, at least five, or at least six, ankyrin repeat domains. In some embodiments, the protein of the invention comprises more than one, e.g., at least two, at least three, at least four, at least five, or at least six, ankyrin repeat domains each corresponding to an ankyrin repeat domain of the invention. In some embodiments, the protein of the invention comprises only one ankyrin repeat domain.

In some embodiments, the protein of the invention is multivalent, i.e. it comprises multiple identical moieties binding to the same target, in particular multiple identical ankyrin repeat domains binding to the same target. In some embodiments, the protein is bivalent, trivalent, tetravalent, pentavalent or hexavalent. In some embodiments, the protein of the invention is multiparatopic, i.e. it comprises multiple different moieties binding to the same target, in particular multiple different ankyrin repeat domains binding to the same target. In some embodiments, the protein is biparatopic, triparatopic, tetraparatopic, pentaparatopic or hexaparatopic. In some embodiments, the protein of the invention is multispecific, i.e. it comprises multiple different moieties binding to different targets, in particular multiple different ankyrin repeat domains binding to different targets. In some embodiments, the protein is bispecific, trispecific, tetraspecific, pentaspecific or hexaspecific. In some embodiments, the multivalent, multiparatopic or multispecific protein has ankyrin repeat domains that are all ankyrin repeat domains of the invention. In some embodiments, the multivalent, multiparatopic or multispecific protein has ankyrin repeat domains that all share the same N-terminal capping module of the invention.

The various moieties of the protein, including said ankyrin repeat domain of the invention, may connect covalently and/or non-covalently to one another. The various moieties may connect covalently to one another, for instance, via a peptide linker or via a maleimide-containing crosslinker. Suitable peptide linkers include glycine-serine linkers and proline-threonine linkers. In some embodiments, the suitable peptide linker is a naturally found peptide linker, such as the IgG hinge region. In some embodiments, the peptide linkers have a length of 2 to 24 amino acid residues or 2 to 16 amino acid residues. Exemplary peptide linkers include the linkers of SEQ ID NOs: 98 to 100. The various moieties may also connect non-covalently to one another, for instance, via a multimerization moiety. In some embodiments, a multimerization moiety is an immunoglobulin heavy chain constant region, a leucine zipper or a free thiol which can form a disulfide bond with another free thiol.

In some embodiments, the protein comprises one or more additional ankyrin repeat domains as further moieties that are connected by a proline-threonine linker.

The ankyrin repeat domain of the invention may be derived from various methods, such as selection from a protein library, *in silico* design or by mutating an existing ankyrin repeat domain. Subsequently, the protein comprising the ankyrin repeat domain of the invention (and possibly one or more further moieties) may be expressed or synthesized by methods known in the art and, e.g., formulated as a pharmaceutical product.

Accordingly, in a further aspect, the present disclosure relates to a library of proteins comprising an ankyrin repeat domain and including the protein of the invention. In some embodiments, the protein library comprises at least 10³, at least 10⁵, at least 10⁷, at least 10⁹, at least 10¹¹, at least 10¹² or at least 10¹³ proteins. In some embodiments, the protein library comprises at least 10³, at least 10⁵, at least 10⁷, at least 10⁹, at least 10¹¹, at least 10¹² or at least 10¹³ proteins of the invention. In some embodiments, the protein library comprises at least 10³, at least 10⁵, at least 10⁷, at least 10⁹, at least 10¹¹, at least 10¹² or at least 10¹³ proteins that differ in the amino acid sequence of their ankyrin repeat domain. In some embodiments, the protein library comprises at least 10³, at least 10⁵, at least 10⁷, at least 10⁹, at least 10¹¹, at least 10¹² or at least 10¹³ proteins of the invention that differ in the amino acid sequence of their ankyrin repeat domain. In some embodiments, substantially all proteins of the protein library differ in the amino acid sequence of their ankyrin repeat domain. In some embodiments, the protein library exclusively comprises proteins of the invention. In some embodiments, the protein library comprises at least one protein of the invention.

In some embodiments, the protein library comprises proteins having ankyrin repeat domains with different structures. For instance, the protein library may contain a mixture of proteins comprising N2C and N3C ankyrin repeat domains. In some embodiments, the structure of the ankyrin repeat domain is identical for all proteins of the library, e.g., the ankyrin repeat domain of all proteins is either exclusively of N2C structure or exclusively of N3C structure. In some embodiments, the ankyrin repeat domain of all proteins is of the N2C structure. In other embodiments, the ankyrin repeat domain of all proteins is of the N3C structure. In some embodiments, the proteins of the library each comprise a single ankyrin repeat domain only.

The sequence variability in the ankyrin repeat domains of the protein library may be brought about randomly, e.g., by error-prone PCR of the nucleic acid molecules encoding the proteins, or it may be obtained by rational design followed by, e.g., direct synthesis of the nucleic acid molecules encoding the proteins ("design approach"). In some embodiments, the variability is introduced by the design approach. In the design approach, variability of the amino acid sequence is introduced in one or more than one position of the ankyrin repeat domains. The variable positions that may be occupied by different amino acid residues are also referred to as "randomized positions", whereas the positions that are always occupied by the same amino acid residue are referred to as "fixed positions". In some embodiments, the randomized positions are those positions occupied by potential target interaction residues and/or the fixed positions are those positions occupied by framework residues. In some embodiments, one or more of the positions occupied by potential target interaction residues are randomized positions. In some embodiments, all positions occupied by potential target interaction residues are randomized positions.

In certain embodiments, there are corresponding fixed positions and randomized positions in the different proteins of the protein library. Due to the intended variability in the randomized positions, the amino acid residues in corresponding randomized position may differ, although there may also be identical amino acid residues in corresponding randomized positions for at least some of the proteins in the library (though, in such cases, the proteins will not necessarily have identical amino acid residues in each of their corresponding randomized positions). In some embodiments, the fixed positions and the randomized positions are the same for the ankyrin repeat domains of each protein of the protein library. In some embodiments wherein the ankyrin repeat domains have multiple internal ankyrin repeats, the internal ankyrin repeats of each ankyrin repeat domain have different randomized and fixed positions. In some embodiments wherein the ankyrin repeat domains have multiple internal ankyrin repeats, the internal ankyrin repeats of each ankyrin repeat domain have different randomized and fixed positions and the fixed positions and the randomized positions are the same for the ankyrin repeat domains of each protein of the protein library. In some embodiments wherein the ankyrin repeat domains have multiple internal ankyrin repeats, the internal ankyrin repeats of each ankyrin repeat domain have the same randomized and fixed positions. In some embodiments wherein the ankyrin repeat domains have multiple internal ankyrin repeats, the internal ankyrin repeats of each ankyrin repeat domain have the same randomized and fixed positions and the fixed positions and the randomized positions are the same for the ankyrin repeat domains of each protein of the protein library.

The randomized positions may show different degrees of variability, i.e. they may be occupied by different sets of amino acid residues. In some embodiments, the degree of variability differs between randomized positions. In some embodiments, the amino acid residue in a randomized position is any of the naturally occurring amino acid residues. In some embodiments, a randomized position may only be occupied by a subset of the naturally occurring amino acid residues. Such subsets can be those having common physicochemical properties, such as sets of hydrophobic, hydrophilic, acidic, basic, aromatic, or aliphatic amino acid residues. Other subsets are those comprising all naturally occurring amino acid residues except for certain non-desired amino acid residues, such as sets not comprising cysteines or prolines. In yet other embodiments, the subsets comprise those amino acid residues that are found in the corresponding positions of naturally occurring ankyrin repeats.

In some embodiments, the proteins of the protein library share at least 70%, at least 75%, at least 80%, at least 85%, at least 90% or at least 95% sequence identity in the amino acid sequence of their ankyrin repeat domains.

The above protein library can serve to select those proteins of the library that have a predetermined property, i.e. a certain property of interest that may be found in the ankyrin repeat domain of one of the proteins of the protein library and that can be screened for. Such predetermined property may include the specific binding to a target, the activation or inhibition of a target, such as an enzyme, and the blocking of an interaction between two targets. In some embodiments, the predetermined property is the specific binding to a target. Preferably, the protein selected from the library is a protein of the invention.

In one embodiment, the present disclosure provides a method for selecting a protein that specifically binds to a target, comprising the following steps:
a) providing a library of proteins comprising an ankyrin repeat domain; and
b) selecting a protein specifically binding to the target via its ankyrin repeat domain from the library,
wherein the selected protein is a protein of the invention.

In one embodiment, the present disclosure provides a method for selecting a protein that specifically binds to a target, comprising the following steps:
a) providing a library of proteins of the invention; and
b) selecting a protein specifically binding to the target via its ankyrin repeat domain from the library.

During the selection step b), the proteins can be selected using screening methods commonly known to the person skilled in the art, such as yeast display, protein fragment complementation assay, phage display or ribosome display. The protein may also be selected during selection step b) by screening the library of step a) *in silico.* In some embodiments, the proteins are selected in step b) using phage display or ribosome display.

As indicated above, the protein of the invention as found in the protein library or represented by the protein selected from the library is not particularly limited in the sequence of the ankyrin repeat domain outside position 24 of the N-terminal capping module. In some embodiments, the N-terminal capping module of such ankyrin repeat domain comprises an amino acid sequence that is substantially identical to an amino acid sequence selected from the group consisting of SEQ ID NOs: 1 to 38, such as an amino acid sequence selected from the group consisting of SEQ ID NOs: 1 to 37. In some embodiments, the thermostability of such ankyrin repeat domain is improved in comparison to a reference ankyrin repeat domain having the same amino acid sequence except for the amino acid residue at position 24 of the N-terminal capping module, which is, e.g., A in the reference ankyrin repeat domain.

After the selection of a protein, the protein can be further modified, mutated and/or optimized by methods commonly known in the art.

For instance, amino acid sequence variants of the protein can be generated, e.g., by subjecting the nucleic acid encoding the selected protein to physical or chemical mutagens, copying said nucleic acid by error-prone PCR, using said nucleic acid for DNA shuffling or random chimeragenesis (Neylon C., Nucleic Acids Res., 32(4), 1448-1459, 2004). The protein library of such amino acid sequence variants may then again be subjected to the above selection step b) in order to select the variant(s) having the predetermined property.

The protein selected in step b) above may also be selectively mutated. For instance, one or more cysteine residues may be introduced, the thiol group(s) of which can then react with maleimide cross-linkers. Similarly, certain non-desirable amino acid residues may be removed, for instance, cysteines, which are prone to oxidations. Also, amino acid residues may be selectively mutated after analysis of the crystal structure so that the protein structure better fits to the target.

The protein selected in step b) may also become modified with one or more further moieties as outlined above for the protein of the invention. In one embodiment, the protein selected in step b) is modified with one or more further ankyrin repeat domains.

In one embodiment, the present disclosure provides a method of modifying a protein comprising an ankyrin repeat domain, e.g., one with an N-terminal capping module that has an amino acid residue other than L at position 24, by replacing the amino acid residue at position 24 of the N-terminal capping module to result in a protein of the invention. By modifying an ankyrin repeat domain in this way, the favorable properties related to the N-terminal capping module disclosed herein may be transferred to the ankyrin repeat domain of the thus obtained protein. In some embodiments, the amino acid residue at position 24 of the N-terminal capping module is replaced alone. In other embodiments, the amino acid residue at position 24 of the N-terminal capping module is replaced together with other amino acid residues, e.g., other amino acid residues of the N-terminal capping module. In some embodiments, one or more of the further mutations referred to above are introduced by replacing the amino acid residue(s) at the corresponding position(s). In some embodiments, the entire N-terminal capping module is replaced. In some embodiments, a sequence substantially identical to any one of SEQ ID NOs: 1 to 38 and including L at position 24 is used to replace the corresponding sequence in the N-terminal capping module.

Thus, in one embodiment, the present disclosure provides a method of preparing a protein comprising the following steps:
a) selecting a protein comprising an ankyrin repeat domain with an N-terminal capping module that does not have L at position 24; and
b) replacing one or more amino acid residues of the protein to result in a protein of the invention.

In one embodiment, the present disclosure provides a method of improving the thermostability of an ankyrin repeat domain comprising the following steps:
a) selecting a protein comprising an ankyrin repeat domain with an N-terminal capping module that does not have L at position 24; and
b) replacing one or more amino acid residues of the protein to result in a protein of the invention, wherein the thermostability of the ankyrin repeat domain of the resulting protein is improved as compared to the ankyrin repeat domain without the replacement of the amino acid residue(s).

As indicated above, a protein of the invention resulting from the replacement method is not particularly limited in the sequence of the ankyrin repeat domain outside position 24 of the N-terminal capping module. In some embodiments, the N-terminal capping module of the ankyrin repeat domain resulting from the replacement method comprises an amino acid sequence that is substantially identical to an amino acid sequence selected from the group consisting of SEQ ID NOs: 1 to 38, such as an amino acid sequence selected from the group consisting of SEQ ID NOs: 1 to 37. In some embodiments, the thermostability of the ankyrin repeat domain resulting from the replacement method is improved in comparison to a reference ankyrin repeat domain having the same amino acid sequence except for the amino acid residue at position 24 of the N-terminal capping module, which is, e.g., A in the reference ankyrin repeat domain. In some embodiments, the thermostability of the ankyrin repeat domain of the protein resulting from the replacement method is improved in comparison to the ankyrin repeat domain of the original protein.

The protein resulting from the replacement method can be further modified, mutated and/or optimized by methods commonly known in the art. In some embodiments, the protein resulting from the replacement method comprises one or more further moieties in addition to the ankyrin repeat domain as outlined above for the protein of the invention. Such modification with one or more further moieties may occur before, during or after the replacement of the one or more amino acid residues. In some embodiments, the one or more further moieties are added to the protein after replacement of the one or more amino acid residues. In some embodiments, the one or more further moieties were added to the protein before replacement of the one or more amino acid residues.

The present disclosure also relates to a method of designing or optimizing the amino acid sequence of the ankyrin repeat domain of the protein of the invention *in silico* through computational methods. It is to be understood that the ankyrin repeat domain may be entirely designed *in silico* or partially, e.g., by optimizing a pre-existing ankyrin repeat domain through computational methods.

Thus, in one embodiment, the present disclosure provides a method of designing a protein comprising designing or optimizing the amino acid sequence of an ankyrin repeat domain *in silico* through computational methods to result in a protein of the invention.

As indicated above, a protein of the invention resulting from such design method is not particularly limited in the sequence of said ankyrin repeat domain outside position 24 of the N-terminal capping module. In some embodiments, the *in silico* designed or optimized ankyrin repeat domain comprises an amino acid sequence that is substantially identical to an amino acid sequence selected from the group consisting of SEQ ID NOs: 1 to 38, such as an amino acid sequence selected from the group consisting of SEQ ID NOs: 1 to 37. In some embodiments, the thermostability of the designed or optimized ankyrin repeat domain is improved in comparison to a reference ankyrin repeat domain having the same amino acid sequence except for the amino acid residue at position 24 of the N-terminal capping module, which is, e.g., A in the reference ankyrin repeat domain.

The protein comprising the designed or optimized ankyrin repeat domain can be further modified, mutated and/or optimized by methods commonly known in the art. In some embodiments, the protein comprising the designed or optimized ankyrin repeat domain comprises one or more further moieties in addition to the ankyrin repeat domain as outlined above for the protein of the invention. Such modification with one or more further moieties may occur before, during or after the *in silico* design or optimization of the ankyrin repeat domain.

In some embodiments, the protein of the invention, e.g., a protein resulting from one of the above methods, is expressed or synthesized. In some embodiments, the expressed or synthesized protein is purified after its expression or synthesis. In some embodiments, the expressed or synthesized and, optionally, purified protein is formulated as a pharmaceutical composition.

In further aspects, the present disclosure provides a nucleic acid encoding the protein of the invention, a chromosome or vector comprising such nucleic acid, such as a bacterial or a viral vector, and a cell or *in vitro* expression system comprising such nucleic acid, chromosome or vector.

The nucleic acid can be DNA or RNA, single stranded or double-stranded, in isolated form or part of a larger nucleic acid, e.g., of a vector or a chromosome. The nucleic acid may comprise elements that enable delivery of the nucleic acid to a cell and/or expression of the nucleic acid in a cell. For instance, the nucleic acid encoding the protein of the invention can be operatively linked to expression control sequences, which have an impact on the transcription and/or translation of the protein, such as promoters, enhancers, transcription terminators, start codons and stop codons. Depending on the intended application and/or context, the expression control sequences may be selected from any eukaryotic or prokaryotic organism. Suitable promoters may be constitutive or inducible promoters. Examples include the CMV-, lacZ-, T7-, T5-, RSV-, SV40-, AOX1-, and GAPDH-promoter. Suitable enhancers include the CMV-enhancer, insulin-responsive elements, and SV40-enhancer. Suitable transcription terminators include the SV40-, lacZ-, and tk-polyadenylation signal.

The present disclosure also provides a library of nucleic acids comprising a nucleic acid encoding the protein of the invention. In some embodiments, the nucleic acid library comprises at least 10³, at least 10⁵, at least 10⁷, at least 10⁹, at least 10¹¹, at least 10¹² or at least 10¹³ nucleic acids. In some embodiments, the nucleic acid library comprises at least 10³, at least 10⁵, at least 10⁷, at least 10⁹, at least 10¹¹, at least 10¹² or at least 10¹³ nucleic acids that encode a protein of the invention. In some embodiments, the nucleic acid library comprises at least 10³, at least 10⁵, at least 10⁷, at least 10⁹, at least 10¹¹, at least 10¹² or at least 10¹³ nucleic acids that differ in the amino acid sequence of their ankyrin repeat domain. In some embodiments, the nucleic acid library comprises at least 10³, at least 10⁵, at least 10⁷, at least 10⁹, at least 10¹¹, at least 10¹² or at least 10¹³ nucleic acids that encode a protein of the invention and differ in the amino acid sequence of their ankyrin repeat domain. In some embodiments, substantially all nucleic acids of the library differ in the amino acid sequence of their ankyrin repeat domain. In some embodiments, the nucleic acid library exclusively comprises nucleic acids of the invention. In some embodiments, the nucleic acid library comprises at least one nucleic acid encoding a protein of the invention.

The cell comprising the nucleic acid, the chromosome or the vector of the invention can be a prokaryotic or a eukaryotic cell. In some embodiments, the cell is a bacterial, yeast or mammalian cell. In some embodiments, the cell is derived from *E. coli*, *P. pastoris*, *S. cerevisiae*, human, hamster or mouse. In some embodiments, the cell is selected from CHO, HEK293, BHK, NS0, Sp2/0, HT-1080, PER.C6, CAP and HuH-7 cells.

In some embodiments, the *in vitro* expression system comprising the nucleic acid, chromosome or vector of the invention is based on a cell-free extract from *E. coli*, yeast, rabbit, wheat germ, insect or human.

In a further aspect, the present disclosure provides a method of preparing a protein comprising the following steps:
a) culturing a cell comprising a nucleic acid encoding the protein of the invention under conditions allowing expression thereof; and
b) purifying the expressed protein.

In one embodiment, the present disclosure provides a method of preparing a protein comprising the following steps:
a) assembling by genetic means one or more gene(s) encoding the protein of the invention, wherein one gene comprises a sequence encoding the ankyrin repeat domain that comprises the N-terminal capping module, followed by one or more internal ankyrin repeats and a C-terminal capping module, and
b) expressing the gene(s) encoding the protein of the invention.

The present disclosure also provides a pharmaceutical composition comprising the protein of the invention, the nucleic acid of the invention or the cell of the invention. In some embodiments, the pharmaceutical composition comprises an aqueous solution. For instance, it may comprise at least 1 wt% water. In some embodiments, the pharmaceutical composition is comprised in a glass or a plastic container.

In a further aspect, the present disclosure provides the use of the protein of the invention, the nucleic acid of the invention or the cell of the invention in a method of treating a disease, condition or symptom. In a further aspect, the present disclosure provides a method of treating a disease, condition or symptom comprising the administration of the protein of the invention, the nucleic acid of the invention or the cell of the invention. In a further aspect, the present disclosure provides the use of the protein of the invention, the nucleic acid of the invention or the cell of the invention in the manufacture of a medicament for the treatment of a disease, condition or symptom. In some embodiments, the disease, condition or symptom is selected from the group consisting of cancer, an immunological disease, such as an autoimmune disease, a fibrotic disease, an inflammatory disease, an ophthalmological disease, a neurodegenerative disease, an infectious disease, a nephropathy, a cardiovascular disease or a metabolic disease.

### Examples

### Example 1: Effect of mutations in the N-terminal capping module on the thermostability of the ankyrin repeat domain

Based on crystal structure data, each position of the N-terminal capping module of an ankyrin repeat domain was analyzed and predictions were made about the most suitable amino acid residues for each position. In light of the inherent difficulty to correctly predict the role of the mutations in the N-terminal capping module, various mutations were tested by *in vitro* experimentation.

### Materials and Methods

### Protein sequences

His-tagged ankyrin repeat domains P#58 to P#97 corresponding to SEQ ID NOs: 58 to 97, respectively, were tested.

The DNA sequence encoding each ankyrin repeat domain was chemically synthesized and cloned into pQIq expression vectors (Simon M. et al., Bioconjug Chem., 23(2), 279-86, 2012) by standard techniques.

### Protein expression

The ankyrin repeat domains were expressed in *E. coli* BL21 or XL1-Blue cells and purified via their His-tag using standard protocols. Briefly, 25 ml of stationary overnight cultures (LB, 1% glucose, 100 mg/l of ampicillin; 37°C) were used to inoculate 1 I cultures (same medium). At an absorbance of about 1 at 600 nm, the cultures were induced with 0.5 mM IPTG and incubated at 37°C for 4 h. The cultures were centrifuged and the resulting pellets were resuspended in 40 ml of TBS500 (50 mM Tris-HCl, 500 mM NaCl, pH 8) and sonicated. The lysate was recentrifuged, and glycerol (10% (v/v) final concentration) and imidazole (20 mM final concentration) were added to the resulting supernatant. The ankyrin repeat domains were purified over a Ni-nitrilotriacetic acid column (2.5 ml column volume) according to the manufacturer's instructions (QIAgen, Germany). Up to 200 mg of highly soluble ankyrin repeat domains were purified from one liter of *E. coli* culture with a purity > 95% as estimated from SDS-15% PAGE. Such purified ankyrin repeat domains were used for further characterizations.

### CD measurement

The CD signal of the ankyrin repeat domains was recorded at 222 nm in a Chirascan V100 instrument (Applied Photophysics) while slowly heating the ankyrin repeat domains at a concentration of 0.01 mM in PBS (137 mM NaCl, 10 mM phosphate and 2.7 mM KCl, pH 7.4) from 25°C to 100°C using a temperature ramp of 1°C per min, collecting data periodically at 0.5°C intervals. Since some of the ankyrin repeat domains were particularly stable, the denaturant guanidine chloride was additionally added to the PBS buffer of some of the samples. Specifically, 2M guanidine hydrochloride (GdmCl) was added to the buffer of P#58-P#65, P#78-P#87 and P#92-P#95, 4M GdmCl was added to the buffer of P#66-P#67, P#74-P#77, P#88-P#91 and P#96-P#97 and 6M GdmCl was added to the buffer of P#72-P#73.

Measuring the CD signal of ankyrin repeat domains is an effective means to follow their denaturation as they mainly consist of alpha helices that show a strong change in their CD signal at 222 nm upon unfolding. The midpoint of the observed transition of such a measured CD signal trace for an ankyrin repeat domain corresponds to its Tm value. Tm values were derived as described in V. Consalvi et al. (Protein Eng Des Sel. 13, 501-507, 2000).

### Results and discussion

The melting curves for the above-mentioned ankyrin repeat domains were determined. Based on the measured melting curves, the Tm values of the various constructs were determined as described above.

The influence of position 24 of the N-terminal capping module on thermostability of the ankyrin repeat domain was assessed by comparing P#58 to P#65 that only differ in the amino acid residue at position 24 of their N-terminal capping module (corresponding to position 36 of SEQ ID NOs: 58 to 65).

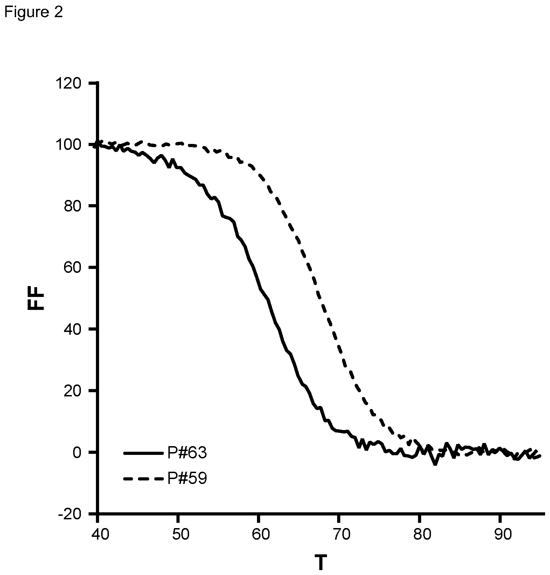

Figure 2 shows, as an example, the melting curves of P#59 and P#63. Table 2 shows the Tm values and the corresponding amino acid at position 24 of the respective N-terminal capping module of P#58 to P#65.

**Table 2:**

| **Protein** | **Position 24** | **Tm value [°C]** |
|---|---|---|
| P#58 | A | 66.0 |
| P#59 | L | 67.6 |
| P#60 | G | 57.9 |
| P#61 | V | 60.0 |
| P#62 | E | 61.9 |
| P#63 | N | 61.3 |
| P#64 | Q | 65.0 |
| P#65 | S | 61.9 |

As reflected by Table 2, the ankyrin repeat domain with the highest Tm value is the one with L at position 24 of the N-terminal capping module, i.e. the ankyrin repeat domain of protein P#59. The increase of the melting temperature is up to about 10°C as compared to the other mutants.

In order to test whether the stabilizing effect of L at position 24 of the N-terminal capping module was specific for the above ankyrin repeat domain or more generally applicable, ankyrin repeat domains with different binding specificities and largely diverging sequences were tested.

Four reference ankyrin repeat domains were used - P#66 that specifically binds to human serum albumin (HSA), P#68 that specifically binds to human vascular endothelial growth factor A (VEGF-A), P#70 that specifically binds to human epidermal growth factor receptor 2 (HER2) and P#72 that specifically binds to the spike protein of SARS-CoV-2. These reference ankyrin repeat domains were then compared to ankyrin repeat domains having the same sequence except for position 24 of the N-terminal capping module, which was mutated to L.

The Tm values of these ankyrin repeat domains are summarized in Table 3:

**Table 3:**

| **Protein** | **Target** | **Position 24** | **Tm value [°C]** |
|---|---|---|---|
| P#66 | HSA | A | 49.0 |
| P#67 | HSA | L | 52.5 |
| P#68 | VEGF-A | N | 63.3 |
| P#69 | VEGF-A | L | 68.0 |
| P#70 | HER2 | N | 66.1 |
| P#71 | HER2 | L | 69.2 |
| P#72 | Spike protein | A | 63.8 |
| P#73 | Spike protein | L | 65.5 |

As reflected by Table 3, the mutation to L at position 24 of the N-terminal capping module resulted in a Tm increase of the ankyrin repeat domain of 1.7 to 4.7°C, thus demonstrating that the increase in thermostability that is caused by L at position 24 of the N-terminal capping module is broadly applicable.

The effect of other mutations in the N-terminal capping module (in combination with L at position 24 or not) were also tested, further demonstrating the suitability of using L at position 24 of the N-terminal capping module. The measured Tm values were as follows: P#74 (Tm of 56.5°C), P#75 (Tm of 68.8°C), P#76 (Tm of 66.3°C), P#77 (Tm of 67.1°C), P#78 (Tm of 63.4°C), P#79 (Tm of 66.0°C), P#80 (Tm of 63.7°C), P#81 (Tm of 64.9°C), P#82 (Tm of 62.6°C), P#83 (Tm of 63.6°C), P#84 (Tm of 63.0°C), P#85 (Tm of 63.7°C), P#86 (Tm of 62.3°C), P#87 (Tm of 64.0°C), P#88 (Tm of 62.0°C), P#89 (Tm of 62.0°C), P#90 (Tm of 61.6°C), P#91 (Tm of 62.5°C), P#92 (Tm of 69.5°C), P#93 (Tm of 70.5°C), P#94 (Tm of 63.0°C), P#95 (Tm of 63.4°C), P#96 (Tm of 62.0°C) and P#97 (Tm of 62.6°C).

## Claims

1. A protein comprising an ankyrin repeat domain,
wherein the ankyrin repeat domain has an N-terminal capping module with L at the position corresponding to position 24 of SEQ ID NO: 1,
wherein said N-terminal capping module comprises an amino acid sequence that has at least 70% sequence identity to an amino acid sequence selected from the group consisting of SEQ ID NOs: 1 to 37, and
wherein said ankyrin repeat domain has a higher melting temperature than a reference ankyrin repeat domain having the same amino acid sequence except for the amino acid residue of the N-terminal capping module at the position corresponding to position 24 of SEQ ID NO: 1, which is A in the reference ankyrin repeat domain.

2. The protein according to claim 1, wherein said N-terminal capping module further has one or more of the following mutations:
(i) an amino acid residue selected from the group consisting of I, T, A, V, L and M at position 15;
(ii) an amino acid residue selected from the group consisting of R and K at position 19;
(iii) an amino acid residue selected from the group consisting of L, V and I at position 22; and
(iv) an amino acid residue selected from the group consisting of A and K at position 23.

3. The protein according to claim 1 or 2, wherein said N-terminal capping module comprises an amino acid sequence at positions 19 to 24 selected from the group consisting of: (R/K)(I/E)L(L/I/M)(A/K)L, RILMAL, RELLKL, RILLKL, RELIKL, RILIKL, RELLAL, RILLAL, RELIAL, RILIAL, KILMAL, KELLKL, KILLKL, KELIKL, KILIKL, KELLAL, KILLAL, KELIAL and KILIAL.

4. The protein according to any one of claims 1 to 3, wherein said protein comprises one or more further ankyrin repeat domains.

5. A nucleic acid comprising a sequence encoding a protein according to any one of claims 1 to 4.

6. A cell comprising the nucleic acid according to claim 5.

7. A library of proteins comprising an ankyrin repeat domain, wherein the library comprises one or more proteins according to any one of claims 1 to 4.

8. A method for selecting a protein that specifically binds to a target comprising the following steps:
(i) providing the library of proteins according to claim 7; and
(ii) selecting a protein from the library that specifically binds to the target via its ankyrin repeat domain wherein the selected protein is a protein according to any one of claims 1 to 4.

9. A method of preparing a protein comprising the following steps (A) or (B):
(A)
(i) selecting a protein comprising an ankyrin repeat domain with an N-terminal capping module that does not have L at the position corresponding to position 24 of SEQ ID NO: 1; and
(ii) replacing one or more amino acid residues of the protein to result in a protein according to any one of claims 1 to 4.
or
(B) designing or optimizing the amino acid sequence of an ankyrin repeat domain *in silico* through computational methods to result in a protein according to any one of claims 1 to 4, wherein in (A) and (B) the ankyrin repeat domain has an N-terminal capping module with L at the position corresponding to position 24 of SEQ ID NO: 1, wherein said N-terminal capping module comprises an amino acid sequence that has at least 70% sequence identity to an amino acid sequence selected from the group consisting of SEQ ID NOs: 1 to 37, and wherein said ankyrin repeat domain has a higher melting temperature than a reference ankyrin repeat domain having the same amino acid sequence except for the amino acid residue of the N-terminal capping module at the position corresponding to position 24 of SEQ ID NO: 1, which is A in the reference ankyrin repeat domain.

10. The method according to claim 8 or 9, wherein the resulting protein is further modified with one or more further moieties and/or with one or more further ankyrin repeat domains.

11. A method of producing a protein comprising the following steps:
(i) expressing or synthesizing a protein according to any one of claims 1 to 4 or a protein resulting from the method according to any one of claims 8 to 10; and
(ii) purifying the expressed or synthesized protein.

12. The method according to claim 11, further comprising the following step:
(ii) formulating the purified protein as a pharmaceutical composition.

13. A protein resulting from the method according to any one of claims 8 to 11.

14. A pharmaceutical composition comprising any one of the following: the protein according to any one of claims 1 to 4 and 13, the nucleic acid according to claim 5 and the cell according to claim 6,
wherein the pharmaceutical composition further comprises a pharmaceutically acceptable carrier.

## Patentansprüche

1. Protein, umfassend eine Ankyrin-Repeat-Domäne,
wobei die Ankyrin-Repeat-Domäne ein N-terminales Capping-Modul mit L an der Position besitzt, die Position 24 von SEQ ID NO: 1 entspricht,
wobei das besagte N-terminale Capping-Modul eine Aminosäuresequenz umfasst, die mindestens zu 70 % identisch ist mit einer Aminosäuresequenz ausgewählt aus der Gruppe bestehend aus SEQ ID NOs: 1 bis 37, und
wobei die besagte Ankyrin-Repeat-Domäne eine höhere Schmelztemperatur hat als eine Referenz-Ankyrin-Repeat-Domäne mit der gleichen Aminosäuresequenz, abgesehen vom Aminosäurerest des N-terminalen Capping-Moduls an der Position, die Position 24 von SEQ ID NO: 1 entspricht, die in der Referenz-Ankyrin-Repeat-Domäne A ist.

2. Protein nach Anspruch 1, wobei das besagte N-terminale Capping-Modul ferner eine oder mehrere der folgenden Mutationen aufweist:
(i) einen Aminosäurerest ausgewählt aus der Gruppe bestehend aus I, T, A, V, L und M an Position 15;
(ii) einen Aminosäurerest ausgewählt aus der Gruppe bestehend aus R und K an Position 19;
(iii) einen Aminosäurerest ausgewählt aus der Gruppe bestehend aus L, V und I an Position 22; und
(iv) einen Aminosäurerest ausgewählt aus der Gruppe bestehend aus A und K an Position 23.

3. Protein nach Anspruch 1 oder 2, wobei das besagte N-terminale Capping-Modul eine Aminosäuresequenz an den Positionen 19 bis 24 umfasst, ausgewählt aus der Gruppe bestehend aus: (R/K)(I/E)L(L/I/M)(A/K)L, RILMAL, RELLKL, RILLKL, RELIKL, RILIKL, RELLAL, RILLAL, RELIAL, RILIAL, KILMAL, KELLKL, KILLKL, KELIKL, KILIKL, KELLAL, KILLAL, KELIAL und KILIAL.

4. Protein nach einem der Ansprüche 1 bis 3, wobei das besagte Protein eine oder mehrere weitere Ankyrin-Repeat-Domänen umfasst.

5. Nukleinsäure, umfassend eine Sequenz, die ein Protein nach einem der Ansprüche 1 bis 4 kodiert.

6. Zelle, umfassend die Nukleinsäure nach Anspruch 5.

7. Protein-Bibliothek, umfassend eine Ankyrin-Repeat-Domäne, wobei die Bibliothek ein oder mehrere Proteine nach einem der Ansprüche 1 bis 4 umfasst.

8. Verfahren zur Auswahl eines Proteins, das speziell an ein Ziel bindet, umfassend die folgenden Schritte:
(i) Bereitstellen der Protein-Bibliothek nach Anspruch 7; und
(ii) Auswählen eines Proteins aus der Bibliothek, das über seine Ankyrin-Repeat-Domäne speziell an das Ziel bindet, wobei das ausgewählte Protein ein Protein nach einem der Ansprüche 1 bis 4 ist.

9. Verfahren zur Herstellung eines Proteins, umfassend die folgenden Schritte (A) oder (B):
(A)
(i) Auswählen eines Proteins, umfassend eine Ankyrin-Repeat-Domäne mit einem N-terminalen Capping-Modul, das an der Position, die Position 24 von SEQ ID NO: 1 entspricht, kein L hat; und
(ii) Ersetzen von einem oder mehreren Aminosäureresten des Proteins zum Erhalt eines Proteins nach einem der Ansprüche 1 bis 4.
oder
(B) Designen oder Optimieren der Aminosäuresequenz einer Ankyrin-Repeat-Domäne *in silico* mittels rechnerischer Verfahren zum Erhalt eines Proteins nach einem der Ansprüche 1 bis 4, wobei in (A) und (B) die Ankyrin-Repeat-Domäne ein N-terminales Capping-Modul mit L an der Position besitzt, die Position 24 von SEQ ID NO: 1 entspricht, wobei das besagte N-terminale Capping-Modul eine Aminosäuresequenz umfasst, die mindestens zu 70 % identisch ist mit einer Aminosäuresequenz ausgewählt aus der Gruppe bestehend aus SEQ ID NOs: 1 bis 37, und wobei die besagte Ankyrin-Repeat-Domäne eine höhere Schmelztemperatur hat als eine Referenz-Ankyrin-Repeat-Domäne mit der gleichen Aminosäuresequenz, abgesehen vom Aminosäurerest des N-terminalen Capping-Moduls an der Position, die Position 24 von SEQ ID NO: 1 entspricht, die in der Referenz-Ankyrin-Repeat-Domäne A ist.

10. Verfahren nach Anspruch 8 oder 9, wobei das resultierende Protein mit einer oder mehreren weiteren Gruppen und/oder mit einer oder mehreren weiteren Ankyrin-Repeat-Domänen weiter modifiziert wird.

11. Verfahren zur Produktion eines Proteins, umfassend die folgenden Schritte:
(i) Exprimieren oder Synthetisieren eines Proteins nach einem der Ansprüche 1 bis 4 oder eines aus dem Verfahren nach einem der Ansprüche 8 bis 10 resultierenden Proteins; und
(ii) Aufreinigen des exprimierten oder synthetisierten Proteins.

12. Verfahren nach Anspruch 11, ferner die folgenden Schritte umfassend:
(ii) Formulieren des aufgereinigten Proteins als pharmazeutische Zusammensetzung.

13. Protein, resultierend aus dem Verfahren nach einem der Ansprüche 8 bis 11.

14. Pharmazeutische Zusammensetzung, umfassend eine der folgenden Komponenten: das Protein nach einem der Ansprüche 1 bis 4 und 13, die Nukleinsäure nach Anspruch 5 und die Zelle nach Anspruch 6,
wobei die pharmazeutische Zusammensetzung ferner einen pharmazeutisch akzeptablen Träger umfasst.

## Revendications

1. Protéine comprenant un domaine de répétition ankyrine,
dans laquelle le domaine de répétition ankyrine présente un module de coiffe N-terminal avec L à la position correspondant à la position 24 de SEQ ID NO : 1,
dans laquelle ledit module de coiffe N-terminal comprend une séquence d'acides aminés présentant une identité de séquence d'au moins 70 % par rapport à une séquence d'acides aminés sélectionnée dans le groupe constitué de SEQ ID NOs : 1 à 37, et
dans laquelle ledit domaine de répétition ankyrine présente une température de fusion plus élevée qu'un domaine de répétition ankyrine de référence présentant la même séquence d'acides aminés, à l'exception du résidu d'acides aminés du module de coiffe N-terminal à la position correspondant à la position 24 de SEQ ID NO : 1, qui est A dans le domaine de répétition ankyrine de référence.

2. Protéine selon la revendication 1, dans laquelle ledit module de coiffe N-terminal présente en outre une ou plusieurs des mutations suivantes :
(i) un résidu d'acides aminés sélectionné dans le groupe constitué de I, T, A, V, L et M à la position 15 ;
(ii) un résidu d'acides aminés sélectionné dans le groupe constitué de R et K à la position 19 ;
(iii) un résidu d'acides aminés sélectionné dans le groupe constitué de L, V et I à la position 22 ; et
(iv) un résidu d'acides aminés sélectionné dans le groupe constitué de A et K à la position 23.

3. Protéine selon la revendication 1 ou 2, dans laquelle ledit module de coiffe N-terminal comprend une séquence d'acides aminés aux positions 19 à 24 sélectionnée dans le groupe constitué de : (R/K)(I/E)L(L/I/M)(A/K)L, RILMAL, RELLKL, RILLKL, RELIKL, RILIKL, RELLAL, RILLAL, RELIAL, RILIAL, KILMAL, KELLKL, KILLKL, KELIKL, KILIKL, KELLAL, KILLAL, KELIAL et KILIAL.

4. Protéine selon l'une quelconque des revendications 1 à 3, dans laquelle ladite protéine comprend un ou plusieurs autres domaines de répétition ankyrine.

5. Acide nucléique comprenant une séquence codant pour une protéine selon l'une quelconque des revendications 1 à 4.

6. Cellule comprenant l'acide nucléique selon la revendication 5.

7. Bibliothèque de protéines comprenant un domaine de répétition ankyrine, dans laquelle la bibliothèque comprend une ou plusieurs protéines selon l'une quelconque des revendications 1 à 4.

8. Méthode de sélection d'une protéine qui se lie spécifiquement à une cible, comprenant les étapes suivantes :
(i) la fourniture de la bibliothèque de protéines selon la revendication 7 ; et
(ii) la sélection d'une protéine parmi la bibliothèque qui se lie spécifiquement à la cible via son domaine de répétition ankyrine, dans laquelle la protéine sélectionnée est une protéine selon l'une quelconque des revendications 1 à 4.

9. Méthode de préparation d'une protéine comprenant les étapes (A) ou (B) suivantes :
(A)
(i) la sélection d'une protéine comprenant un domaine de répétition ankyrine avec un module de coiffe N-terminal qui ne présente pas L à la position correspondant à la position 24 de SEQ ID NO : 1 ; et
(ii) le remplacement d'un ou plusieurs résidus d'acides aminés de la protéine pour résulter en une protéine selon l'une quelconque des revendications 1 à 4.
ou
(B) la conception ou l'optimisation de la séquence d'acides aminés d'un domaine de répétition ankyrine *in silico* au moyen de méthodes informatiques pour résulter en une protéine selon l'une quelconque des revendications 1 à 4, dans laquelle, dans (A) et (B), le domaine de répétition ankyrine présente un module de coiffe N-terminal avec L à la position correspondant à la position 24 de SEQ ID NO : 1, dans lequel ledit module de coiffe N-terminal comprend une séquence d'acides aminés présentant une identité de séquence d'au moins 70 % par rapport à une séquence d'acides aminés sélectionnée dans le groupe constitué de SEQ ID NO : 1 à 37, et dans laquelle ledit domaine de répétition ankyrine présente une température de fusion plus élevée qu'un domaine de répétition ankyrine de référence présentant la même séquence d'acides aminés, à l'exception du résidu d'acides aminés du module de coiffe N-terminal à la position correspondant à la position 24 de SEQ ID NO : 1, qui est A dans le domaine de répétition ankyrine de référence.

10. Méthode selon la revendication 8 ou 9, dans laquelle la protéine résultante est en outre modifiée par un ou plusieurs autres groupements et/ou par un ou plusieurs autres domaines de répétition ankyrine.

11. Méthode de production d'une protéine comprenant les étapes suivantes :
(i) l'expression ou la synthèse d'une protéine selon l'une quelconque des revendications 1 à 4 ou une protéine résultant de la méthode selon l'une quelconque des revendications 8 à 10 ; et
(ii) la purification de la protéine exprimée ou synthétisée.

12. Méthode selon la revendication 11, comprenant en outre l'étape suivante :
(ii) la formulation de la protéine purifiée en tant que composition pharmaceutique.

13. Protéine résultant de la méthode selon l'une quelconque des revendications 8 à 11.

14. Composition pharmaceutique comprenant l'un quelconque des éléments suivants : la protéine selon l'une quelconque des revendications 1 à 4 et 13, l'acide nucléique selon la revendication 5 et la cellule selon la revendication 6,
dans laquelle la composition pharmaceutique comprend en outre un support pharmaceutiquement acceptable.
